# EUROPEAN PATENT SPECIFICATION

(11) **EP 3 432 897 B1**
(45) Date of publication and mention of the grant of the patent: **21.05.2025**
(21) Application number: 17724103.1
(22) Date of filing: 24.03.2017
(51) Int. Cl.: A61K 35/51, A61K 31/7105, C12N 15/113, A61P 17/00

(54) **USE OF UMBILICAL CORD BLOOD DERIVED EXOSOMES FOR TISSUE REPAIR**
VERWENDUNG VON AUS NABELSCHNURBLUT GEWONNENEN EXOSOMEN ZUR GEWEBEREPARATUR
UTILISATION D'EXOSOMES DÉRIVÉS DE SANG DE CORDON POUR LA RÉPARATION TISSULAIRE

(30) Priority: 24.03.2016 US 201662313002 P; 16.05.2016 US 201662336907 P
(43) Date of publication of application: 30.01.2019
(73) Proprietor: Stemlab SA, 3060-197 Cantanhede (PT); Biocant - Associação De Transferência De Tecnologia, Cantanhede 3060-197 (PT)
(72) Inventor: FERREIRA, Lino Da Silva, 3030-076 Coimbra (PT); CORREIA, Joana, Rita, Simoes, 3040-757 Cernache (PT); CARDOSO, Renato, Manuel, Soares, 3050-267 Luso (PT); ANTUNES, Maria, Helena, Henriques, 3030-775 Combra (PT)
(74) Representative: Gata-Goncalves, Ligia
(86) International application number: PCT/IB2017/000412
(87) International publication number: WO 2017/163132

(56) References cited:
- WO-A2-2014/028493
- US-A1- 2012 093 885
- PRANKE P ET AL: "HEMATOLOGIC AND IMMUNOPHENOTYPIC CHARACTERIZATION OF HUMAN UMBILICAL CORD BLOOD", ACTA HAEMATOLOGICA, S. KARGER, BASEL, CH, vol. 105, no. 2, 1 May 2001 (2001-05-01), pages 71 - 76, XP009056147, ISSN: 0001-5792, DOI: 10.1159/000046537
- YEFTA MOENADJAT ET AL: "The application of human umbilical cord blood mononuclear cells in the management of deep partial thickness burn", MED J INDONES, 1 January 2013 (2013-01-01), pages 92 - 99, XP055389174, Retrieved from the Internet <URL:http://mji.ui.ac.id/journal/index.php/mji/article/download/534/521> [retrieved on 20170710]
- HALA O. EL-MESALLAMY ET AL: "Cell-Based Regenerative Strategies for Treatment of Diabetic Skin Wounds, a Comparative Study between Human Umbilical Cord Blood-Mononuclear Cells and Calves' Blood Haemodialysate", PLOS ONE, vol. 9, no. 3, 18 March 2014 (2014-03-18), pages e89853, XP055389176, DOI: 10.1371/journal.pone.0089853
- ZHENG LIU ET AL: "MicroRNA-150 Protects the Heart From Injury by Inhibiting Monocyte Accumulation in a Mouse Model of Acute Myocardial Infarction", CIRCULATION: CARDIOVASCULAR GENETICS, vol. 8, no. 1, 1 February 2015 (2015-02-01), US, pages 11 - 20, XP055389305, ISSN: 1942-325X, DOI: 10.1161/CIRCGENETICS.114.000598
- J.-M. WANG ET AL: "MicroRNA miR-27b Rescues Bone Marrow-Derived Angiogenic Cell Function and Accelerates Wound Healing in Type 2 Diabetes Mellitus", ARTERIOSCLEROSIS, THROMBOSIS, AND VASCULAR BIOLOGY., vol. 34, no. 1, 1 January 2014 (2014-01-01), US, pages 99 - 109, XP055389297, ISSN: 1079-5642, DOI: 10.1161/ATVBAHA.113.302104

## Description

### Technical field

The present patent application refers to a process for preparing a composition comprising exosomes secreted by UCBMNCs, to compositions prepared by this process and to the use of said compositions in the medical area for therapeutical purposes, namely, to treat skin conditions.

### Background of Invention

Chronic wounds are defined as wounds that do not heal in 3 months (Nunan et al., 2014). A wound is a disruption of normal structure and function of the skin. In contrast to acute wounds, chronic wounds fail to proceed through an orderly and timely reparative process that would result in sustained restoration of the skin.

In developed countries, it has been estimated that 1-2 % of the population will experience a chronic wound during their lifetime (Gottrup, 2004). In addition, there are about 150 million diabetes patients worldwide, of which about 15% suffer from foot ulcerations that can evolve into non-healing chronic wounds (Boulton et al., 2005).

Chronic and non-healing wounds are particularly costly because of the need of several treatments in comparison to acute wound treatments. The burden caused by chronic wounds is growing due to an increase of health care costs, aging of the population and a sharp increase of diabetes and obesity worldwide (Sen, 2009).

The most common types of chronic wounds are: (i) arterial ulcers, (ii) venous ulcers, (iii) diabetic ulcers and (iv) pressure ulcers.

Arterial ulcers are formed in patients with hypertension, atherosclerosis and thrombosis, where the reduced blood supply leads to an ischemic state.

Venous ulcers account for more than half of ulcer cases, and they occur mainly in the lower limbs (mainly the legs) and they are associated with deep vein thrombosis, varicose veins and venous hypertension (Eberhardt & Raffetto, 2005). It is estimated that in the United States, there are approximately 0.5 million patients with venous ulcers (Abbade & Lastoria, 2005).

Diabetic ulcers occur in individuals with diabetes mellitus, who have impaired immune function, ischemia (due to poor blood circulation), and neuropathy (nerve damage). Diabetic neuropathy increases the chance of lower-extremity amputations unless treated. In 2004, about 71,000 non-traumatic lower-limb amputations were performed in people with diabetes (Sen, 2009) and the amputations rates increase with age. Pressure ulcers occur due to a constant pressure and friction from body weight over a localized area which may lead to breakage of skin and ulceration. Vulnerable patients include aged people, stroke victims, patients with diabetes or dementia, patients with spinal cord injury, patients in wheelchairs or suffering from impaired mobility or sensation. Annually, 2.5 million pressure ulcers are treated in the United States in acute care facilities alone (Reddy et al., 2006).

### The Wound Healing Process

Wound healing is a dynamic process that consists of four continuous and overlapping phases: haemostasis, inflammation, proliferation, and tissue remodelling. (Guo et al., 2010). For optimal wound healing, events during each phase must occur in a precise and regulated manner. Interruptions, aberrancies, or prolongation in the process can lead to delayed wound healing or non-healing chronic wounds. (Guo et al., 2010).

The haemostasis phase of wound healing begins immediately after injury and is characterized by vascular constriction, platelet aggregation, degranulation, and fibrin clot formation. (Guo et al, 2010). Specifically, contact with exposed extracellular matrix at the site of injury causes platelets to release clotting factors, leading to the formation of a blood clot. In addition, the contact will also cause platelets to release pro-inflammatory cytokines and growth factors such as transforming growth factor (TGF)-β, platelet derived growth factor (PDGF), fibroblast growth factor (FGF), epidermal growth factor (EGF), and insulin-like growth factor (IGF). These molecules activate and attract neutrophils and macrophages thereby initiating the inflammatory phase of wound healing. These molecules also attract endothelial cells and fibroblasts, which will affect the later phases of proliferation and tissue remodelling. (Guo et al., 2010; Velnar et al., 2009).

The inflammatory phase of wound healing is characterized by neutrophil infiltration, monocyte infiltration and differentiation into macrophage, and lymphocyte infiltration.

Neutrophils primarily function is to clear, via phagocytosis, invading microbes and cellular debris in the wound site by producing substances such as proteases and reactive oxygen species (ROS). Macrophages carry out several functions in the wound healing process, including releasing cytokines to recruit and activate leukocytes and clearing apoptotic cells (including neutrophils). Macrophages also release growth factors such as TGF-β and FGF, which activates keratinocytes, fibroblasts, and endothelial cells thereby initiating the proliferative phase of wound healing. Lymphocytes also migrate into wounds during late inflammatory phase. However, their role wound healing is not completely understood. (Guo et al., 2010; Velnar et al., 2009).

The proliferative phase is characterized by re-epithelialization, angiogenesis, collagen synthesis, and extracellular matrix formation. In the reparative dermis, fibroblasts and endothelial cells are the most prominent cell types present and they function to support capillary growth, collagen formation, and the formation of granulation tissue at the site of injury. (Guo et al, 2010; Velnar et al., 2009).

The remodelling phase is the final phase of wound healing, and it may last up to one or two years, sometimes for an even more prolonged period of time. The remodelling phase is a delicate balance between degradation and synthesis and is characterized by collagen remodelling and vascular maturation and regression. Specifically, the extracellular matrix is remodelled into an architecture which approaches that of the normal tissue, and the wound undergoes contraction mediated by myofibroblasts. (Guo et al., 2010; Velnar et al., 2009).

### Diabetes Mellitus

Diabetes mellitus represents a spectrum of metabolic disorders characterized by chronic hyperglycaemia. Type I diabetes mellitus, also known as juvenile-onset diabetes, results from the autoimmune destruction of insulin-secreting pancreatic β-cells. Type II diabetes mellitus, also known as adult-onset diabetes, is characterized by excessive insulin secretion, tissue insulin resistance, and subsequent β-cell dysfunction. (Goutos et al., 2015).

### Effect of Diabetes Mellitus on the Wound Healing Process

Diabetic individuals exhibit impaired healing of acute wounds, and are prone to develop chronic wounds, such as diabetic foot ulcers. (Guo et al., 2010).

Diabetes mellitus exerts a detrimental effect on several aspects of the wound healing process. For example, diabetes alters the balance between different phenotypes of macrophages which causes increased number of inflammatory cells and hinders the transition into the proliferative phase. Diabetes is also associated with reduced levels of growth factors at the wound site, abnormal extracellular matrix deposition, and inhibition of vascularization. (Goutos et al., 2015; Guo et al., 2010).

### Exosomes

Exosomes are liposome-like vesicles (30-200 nm) secreted by most types of cells, which are formed inside the secreting cell in compartments referred as multivesicular bodies (MVBs) and are subsequently released by fusion of the endosomal compartment with the plasma membrane, resulting in content release to the extracellular milieu (Raposo and Stoorvogel, 2013). They contain specific sets of proteins, lipids and RNA, depending on the type of secreting cell and stimuli, and not a random sample of cytoplasmic content (Raposo and Stoorvogel, 2013; Mathivanan S. and Simpson R.J., 2009). They carry genetic material, in the form of mRNA and microRNA, a feature that promoted them to promising biologically derived gene-delivery systems (O'Loughlin et al., 2012).

Cell types employed in experimental regeneration of injured tissues, such as mesenchymal stem cells (MSC) or hematopoietic stem cells (HSC), are rich sources of exosomes, which have been proposed to successfully replace cell-based therapies in different injury models (Bian et al., 2014; Shabbir et al., 2015; Zhang B. et al., 2015; Zhang J. et al.; Lai RC, 2010; Zhou Y, 2013; Sahoo S, 2011).

MSC are multipotent, non-hematopoietic adult stem cells, which can be isolated from umbilical cord (Erices et al., 2000; Gang et al., 2004), placental or adipose tissue, and bone marrow. These cells can differentiate into several cell types such as osteoblasts, chondrocytes, adipocytes and endothelial cells (Pittenger et al., 1999; Crapnell et al., 2013).

It has been recently demonstrated that, in addition to their direct regenerative potential through differentiation, these cells can induce repair by paracrine stimulation, such as exosomes secretion (Lai et al., 2011; Lai et al., 2010). Exosomes secreted by MSC have been shown to induce repair in different mice models of disease, such as myocardial infarction (Lai et al., 2010) and also wound healing (Zhang et al., Stem Cells 2014; Shabir et al., Stem Cells Dev 2015; Zhang et al. J Transl Med 2015).

Despite many papers demonstrating that exosomes derived from MSC can induce tissue regeneration such as wound healing, the same does not hold true for exosomes derived from Umbilical Cord Blood Mononuclear Cells (UCBMNCs).

Furthermore, the bioactivity of exosomes secreted by human Umbilical Cord Blood mononuclear cells has not been assessed, and neither was its therapeutic potential for the treatment of chronic wounds.

Document US2012093885A1 describes compositions comprising UCBMNCs and exosomes secreted by UCBMNCs and a process for preparing said compositions. These compositions are applicable in tissue repair in the scope of regenerative therapies, in particular in angiogenic therapy associated to cardiovascular disease. This document further discloses that cells collected from UBMNCs and respective exosomes, are cultured in a suitable conditioning culture medium comprising namely 0.25% human serum albumin and endothelial-growth factor. It is generally accepted that exosome features and functionality are greatly affected by the type of the cells that secrete them but also by the conditions of processing said cells. In this sense, it is important to note that the process described herein uses (0.1 to 5%) of human serum albumin in the culture medium under conventional O₂ and CO₂ conditions.

Moreover, the exosomes of US2012093885A1 generally present at least two purified molecules selected from the group consisting of miRNA 130a, miRNA 125b, miRNA 92a, miRNA 126, haptoglobin, and hemopexin, cup shape with 30-100 nm in diameter and density of 1.1-1.2 g/cm³ in contrast to the exosome characteristics of the invention. Additionally, said exosomes are secreted by UBMNC CD34+ cells and are used in angiogenic therapy associated to cardiovascular disease as replacement of CD34+ cells.

Additionally, the exosomes described in US2012093885A1 are not suitable to be used in skin regeneration therapies with the aim of increase the survival rate of endothelial cells and keratinocytes, increase the proliferation of endothelial cells, fibroblasts and keratinocytes, and stimulate skin wound healing.

Therefore, there is an urgent need to develop a pharmaceutical composition that can be used in skin regeneration therapies, which is easy to obtain, not expensive, preferably using safe and non-human and animal derived products, conferring an improved degree of efficacy in wound healing and reparation processes even in case of specific and difficult types of wounds such as chronic and non-healing wounds.

The process and exosome compositions of the present invention solve the prior art problems by providing exosome composition comprising exosomes secreted by UCBMNCs conditioning said UCBMNCs by exposure to hypoxic conditions of 0.5% O₂, 5% CO₂, in a culture medium depleted of foetal bovine serum (0% FBS)

### Summary of the Invention

The present invention provides a process of making an exosome composition with exosomes secreted by umbilical cord blood mononuclear cells (UCBMNCs) comprising the following steps:
a) Providing isolated umbilical cord blood mononuclear cells (UCBMNCs),
b) Conditioning the UCBMNCs of step a) by exposure to hypoxic conditions of 0.5% O₂, 5% CO₂, during 18h, in a culture medium depleted of foetal bovine serum (0% FBS),
c)separating and isolating the exosomes secreted by the UCBMNCs of the previous step b), and
d) suspending the isolated exosomes of step c) in a suitable carrier, according to claim 1.

The exosome as prepared by the process as described above, comprises at least one of the exosomes contains one or more miRNA selected from a group consisting of:
- hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, and hsa-miR-101-3p, and
- the exosomes exhibit a spherical morphology,
- the exosomes have an average particle size of 100 nm to 200 nm,
- the exosomes have a negative zeta potential of -10mV to -50mV,
- at least one of the exosomes express the markers CD9, CD34, CD81, and/or Hsc70, and
- the exosomes increase the survival rate of endothelial cells and keratinocytes and stimulate wound healing, according to claim 2.

Disclosed herein but not part of the invention is a process of making a composition comprising miRNA hsa-miR-150-5p and a pharmaceutically acceptable carrier comprising step a) mixing the miRNA hsa-miR-150-5p with a transfection agent, and b) suspending the mixture in a pharmaceutically acceptable carrier.

Pharmaceutical compositions comprising the exosome compositions according to the present invention are advantageously used in skin medical applications such as in skin wound therapies, namely in skin open wound therapy such as an incision, a laceration, a tear, an abrasion, an avulsion or a surgical wound, in burn wounds therapy, such as a thermal burn, a chemical burn or a radiation burn.

### Brief Description of the Drawings

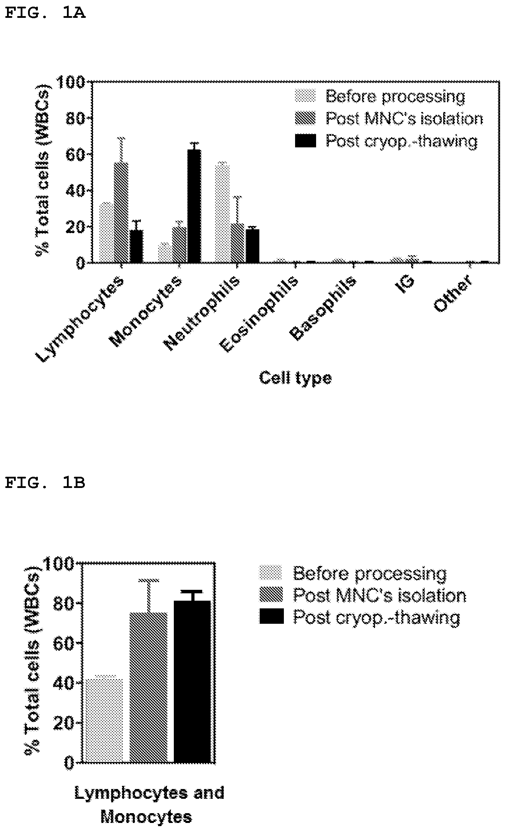

**Figure 1****. Characterization of Umbilical Cord Blood Mononuclear Cells and the impact of cryopreservation, as analysed in the haematological analyser.**
   (A) Detailed composition of the different white blood cells subpopulations present in umbilical cord blood (lymphocytes, monocytes, neutrophils, eosinophils and basophils).
   (B) Impact of processing in the composition of umbilical cord blood mononuclear cells (total lymphocytes and monocytes) relative to the total white blood cells.

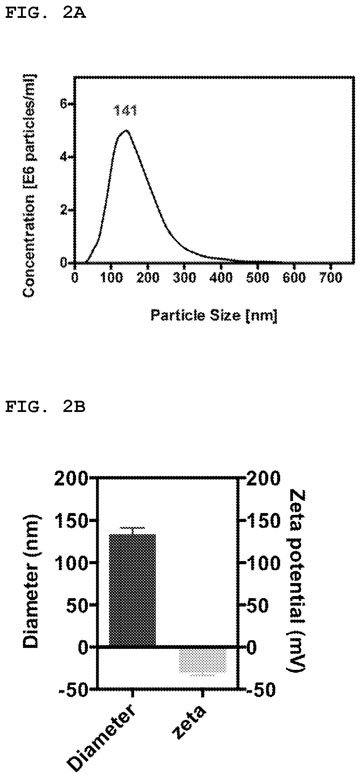

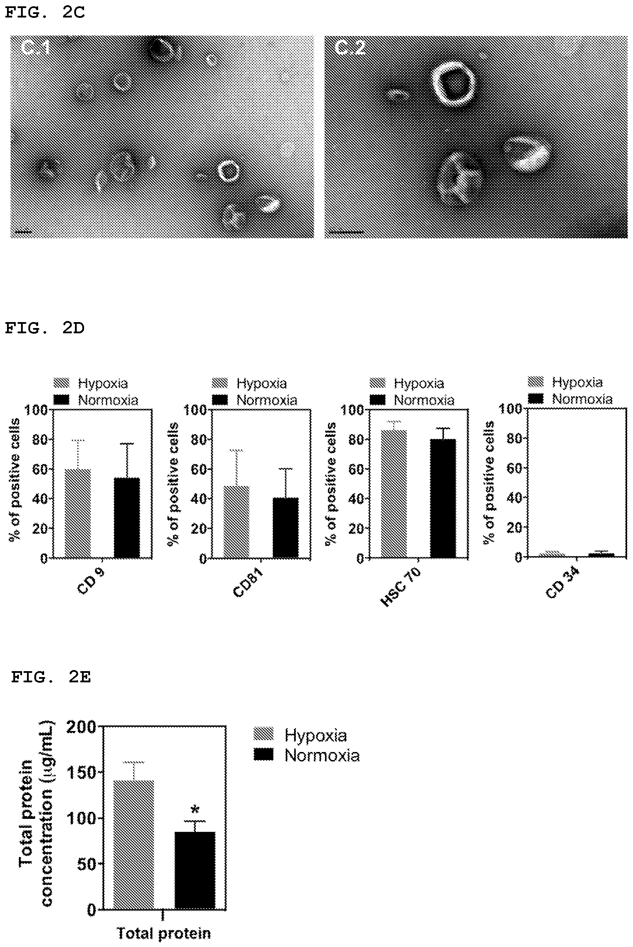

**Figure 2****. Characterization of exosomes secreted by Umbilical Cord Blood Mononuclear Cells.**
   (A) Size distribution of exosomes by DLS.
   (B) Zeta potential of exosomes as evaluated by DLS.
   (C.1) Size and morphology of exosomes secreted by UCBMNCs as evaluated by TEM. (C.2) Size and morphology of exosomes secreted by UCBMNCs as evaluated by TEM.
   (D) Expression of surface markers in exosomes secreted by UCBMNCs preconditioned in hypoxia and in control conditions (normoxia) as analysed by flow cytometry. Results are average +/- SEM, n=3.
   (E) Total protein content, as quantified with the DC protein assay from Biorad of exosomes secreted by UCBMNCs preconditioned in hypoxia (Exo-Hyp) and normoxia (Exo_Nor). Results are average +/-SEM, n=3. Statistical analyses were performed by a t-test. *p<0.05.

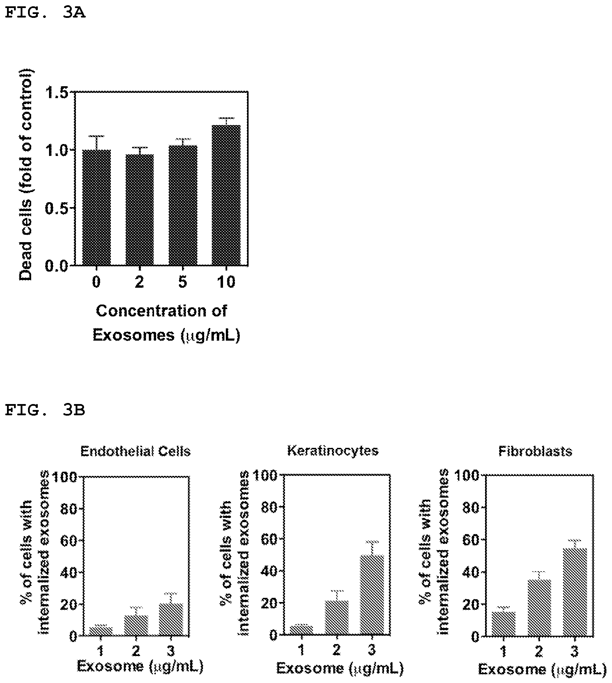

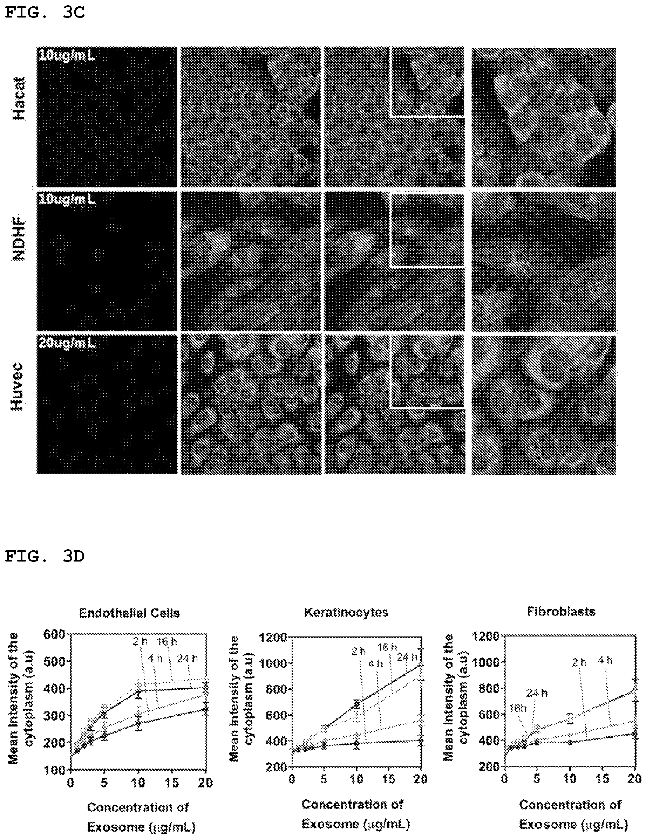

**Figure 3****. Exosome cytotoxicity and exosome uptake in endothelial cells, fibroblasts and keratinocytes.**
   (A) Cytotoxicity of exosomes (from 3 different donors) in HUVECs as evaluated by propidium iodide/Hoechst staining. Cells were exposed to exosomes for 24h and viability assessed after 72h. Results are average +/- SEM, n=3 per donor. Approximately 2000 cells were quantified in each image.
   (B) Uptake of exosomes by endothelial cells, fibroblasts and keratinocytes, as evaluated by flow cytometry. Results are average +/- SEM, n=4-6.
   (C) Confocal microscopy of skin cells exposed to Syto^{®} RNASelect^{™} labelled exosomes (1-20 µg/ml) in exo-depleted medium and incubated for 16 h.
   (D) Quantification of Syto^{®} RNASelect^{™} labelled exosomes in HUVECs, HaCaT cells and fibroblasts. Results are average +/- SEM, n=4.

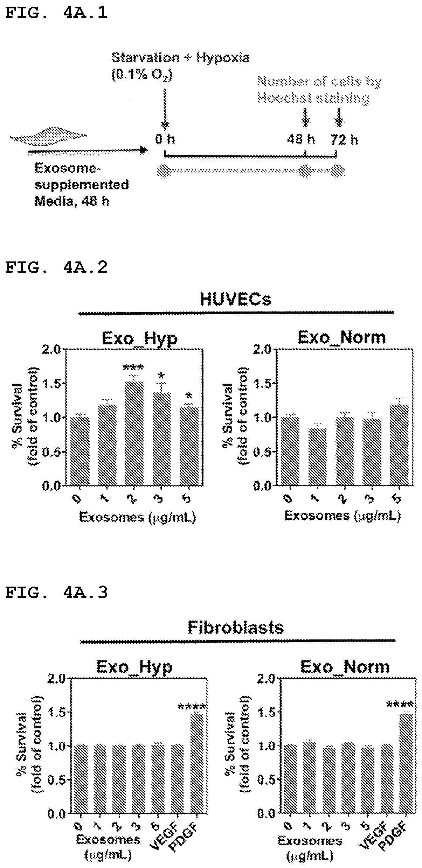

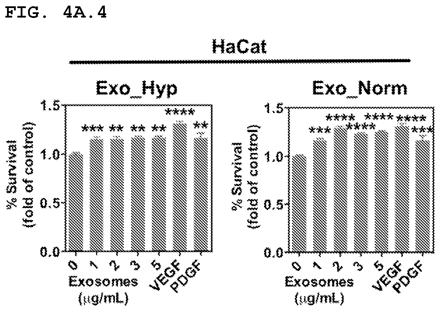

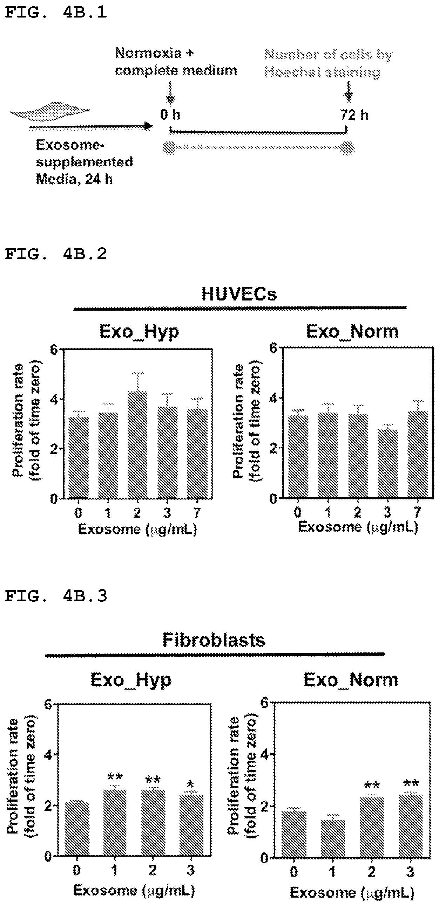

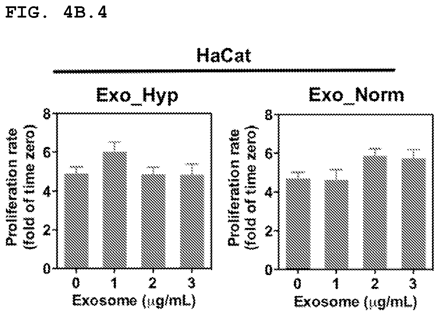

**Figure 4****. Bioactivity UCBMNCs-Exo in skin cells.**
   (A) Exosomes secreted by CD34-depleted UCBMNC cells subjected to ischemic pre-conditioning (Exo_Hyp) increase the survival of endothelial cells and keratinocytes but not fibroblasts. Results were obtained for exosomes collected from 3 different donors. The results are average +/- SEM, n=3 per donor. Vascular endothelial growth factor (VEGF, 50 ng/mL) or platelet derived growth factor (PDGF-BB, 50 ng/mL) were used as controls.
   (B) Proliferation of endothelial cells, fibroblasts and keratinocytes upon treatment with Exo_Hyp or Exo_Nor. Results were obtained for exosomes collected from 3 different donors. The results are average +/- SEM, n=3 per donor. *p<0,05; **p<0,01; ***p<0,001.

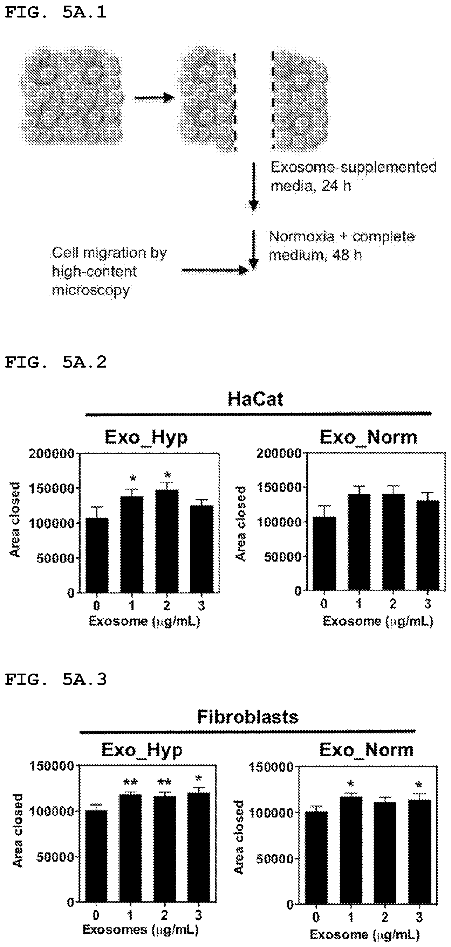

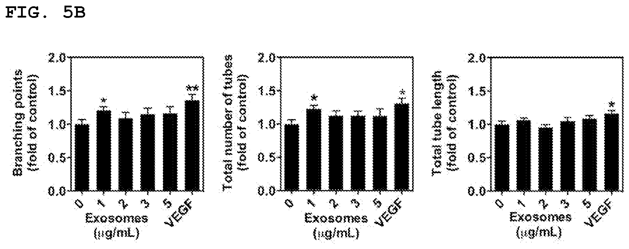

**Figure 5****. Bioactivity UCBMNCs-Exo in skin cells.**
   (A) Scratch assay in keratinocytes and fibroblasts after treatment with Exo_Hyp or Exo_Nor.
   (B) Endothelial tube assay in Matrigel. HUVECs were treated with Exo_Hyp or Exo_Nor.
   In (A) and (B), results were obtained for exosomes collected from 3 different donors. The results are average +/- SEM, n=3 per donor. *p<0,05; **p<0,01; ***p<0,001.

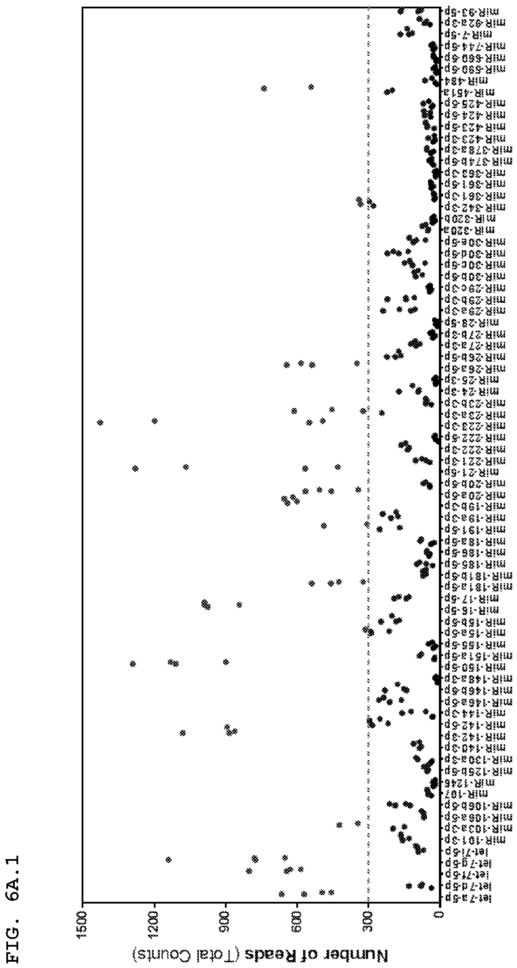

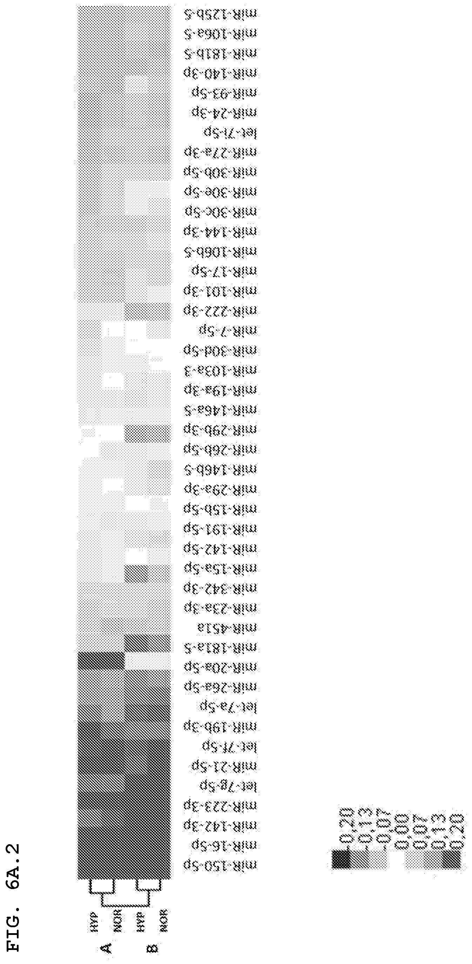

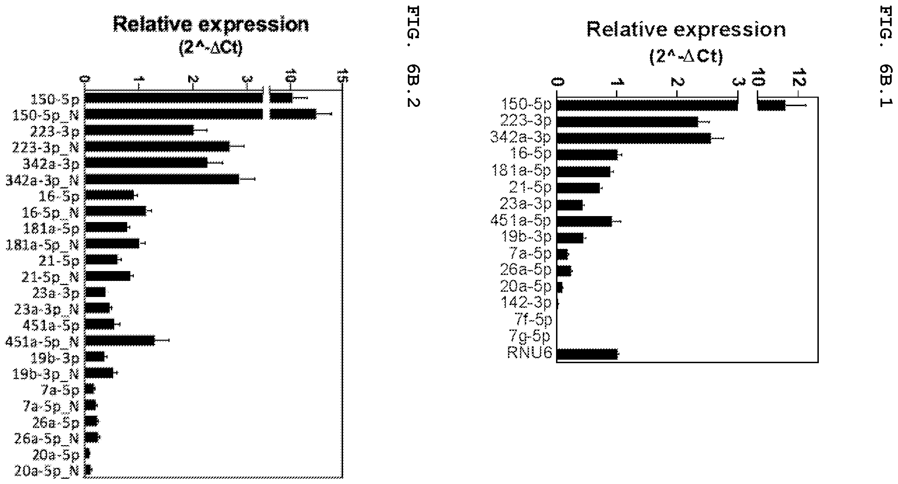

**Figure 6****. Characterization of UCBMNC-Exo miRNA profile.**
   (A) miRNAs identified in Exo_Hyp and Exo_Nor in two different donors.
   (A1) 69 different miRNAs were identified in exosomes secreted by hUCBMNC CD34-depleted from two different donors (A and B) subjected to hypoxia (HYP) and control conditions (NOR) by RNASeq, counting more than 20 reads in all exosomes analysed.
   (A2) Treeview representation of 44 miRs with more than 50 reads for exosomes obtained after conditioning hUCBMNCs of both donors in hypoxia (HYP) and normoxia (NOR).
   (B) Average expression of miRNAs for 10 donors. (B1) Relative expression of the 15 most expressed miRNAs, detected by RNASeq, of exosomes from hypoxia conditioned hUCBMNCs. RNU6 was used as control. Results were obtained for exosomes collected from 10 different donors. Results are average +/- SEM, n=3-4 per donor.
   (B2) Relative expression of 12 most expressed miRNAs of exosomes from hypoxia (HYP) vs normoxia (NOR) conditioned hUCBMNCs. RNU6 was used as control. Results were obtained for exosomes collected from 10 different donors. Results are average +/- SEM, n=3-4 per donor. Statistical analysis was performed by one-way ANOVA with Newman-Keuls multiple comparisons test, *p<0,05; **p<0,01; ***p<0,001.

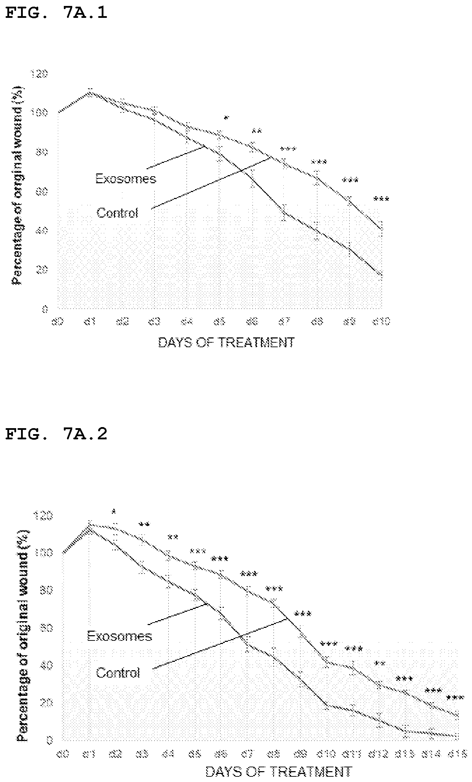

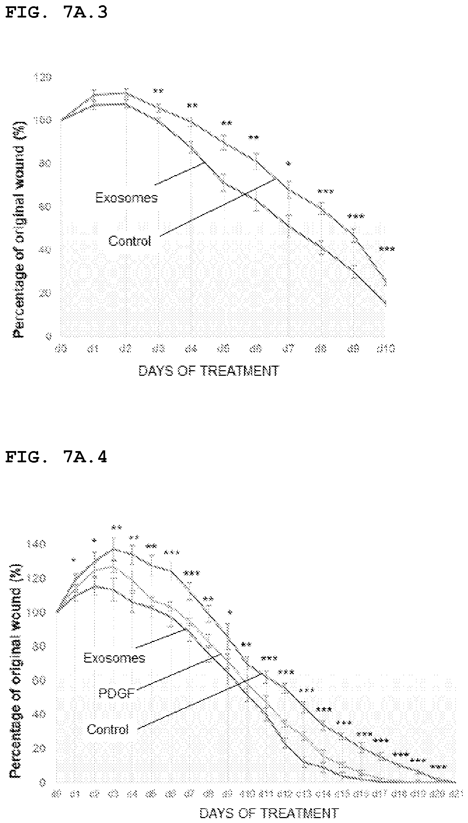

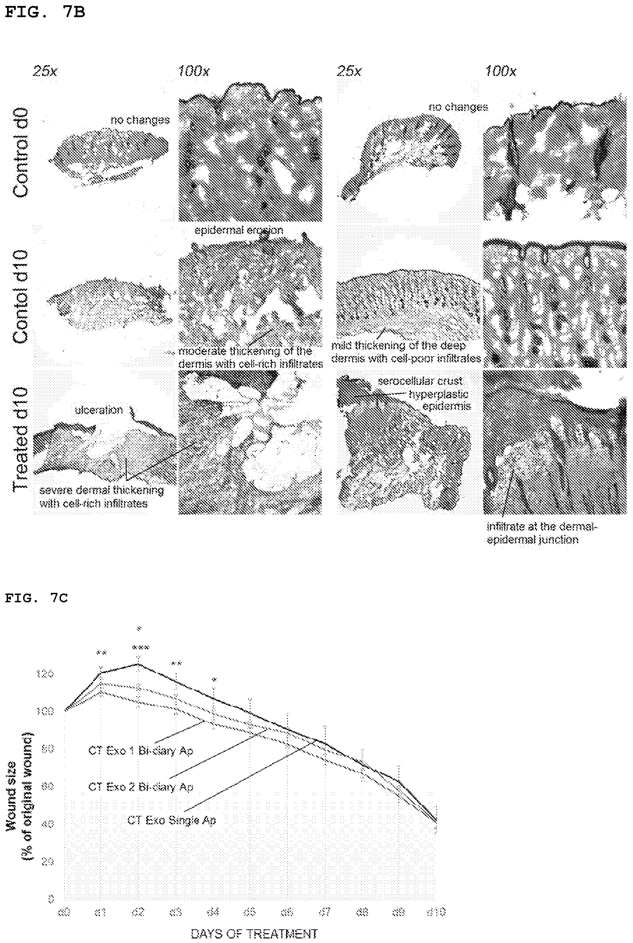

**Figure 7****. UCBMNC-Exo stimulate wound healing in normal and Type I and II diabetic mouse models.**
   (A1) UCBMNC-Exo from Donor 1; (A2) UCBMNC-Exo from Donor 2; (A3) UCBMNC-Exo from Donor 1 administered to non-diabetic animals; (A4) UCBMNC-Exo from Donor 1 administered to genetic diabetic animals (db/db);
   (B) H&E staining of control and treated wounds (diabetes Type 1) at day 10;
   (C) Comparison of the wound closure rates between wounds treated with control saline solution (Control Wound), when UCBMNC-Exo are applied at a distant wound only once on day 1 (CT Exo Single Ap), or twice daily (CT Exo 1 Bi-diary Ap and CT Exo 2 Bi-diary Ap). UCBMNC-Exo applied twice daily to distant excisional wounds stimulate wound healing at the control wound.

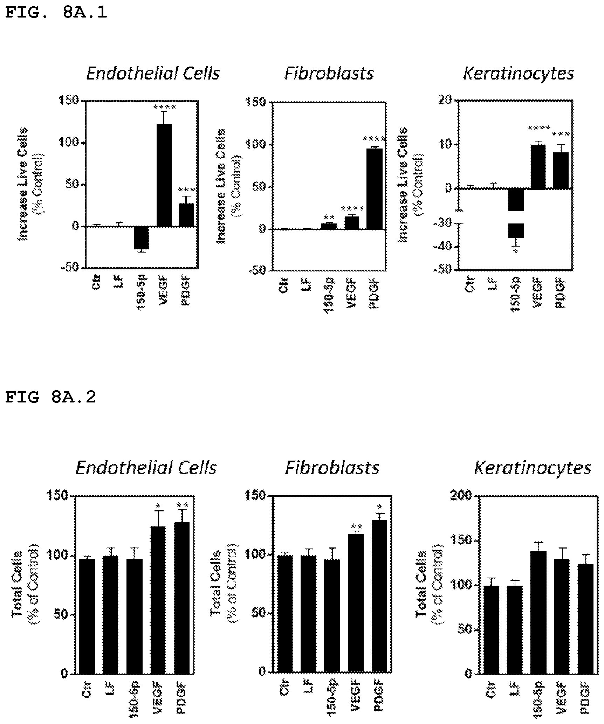

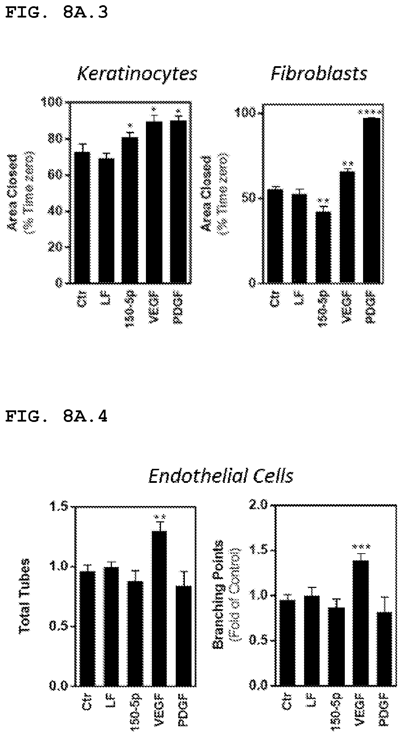

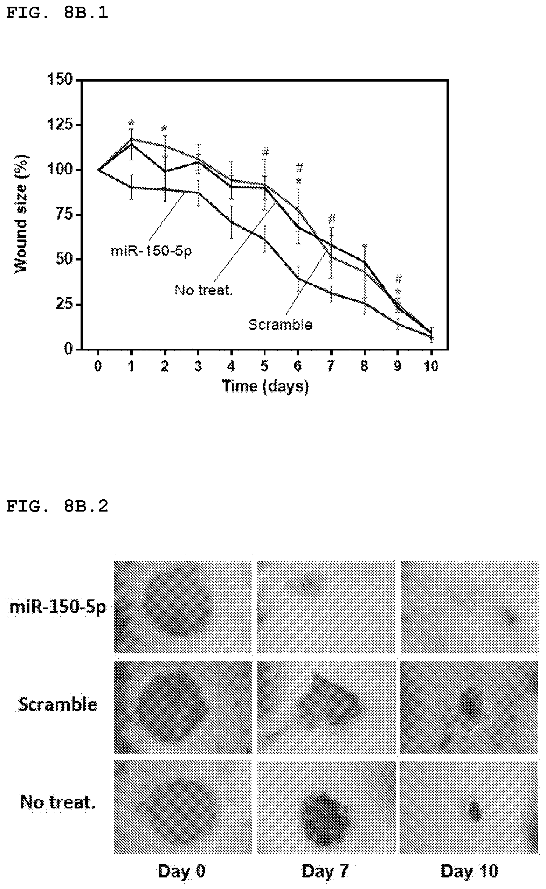

**Figure 8****. MiR-150-5p enhances fibroblasts survival to ischemia and keratinocyte migration *in vitro,* and wound healing *in vivo.***
   (A) *In vitro* effect of miR-150-5p in (A1) Survival in ischemic conditions and (A2) Proliferation of endothelial cells, fibroblasts and keratinocytes, (A3) Scratch assay of fibroblasts and keratinocytes and (A4) tube formation of endothelial cells in Matrigel. Vascular endothelial growth factor (VEGF, 50 ng/mL) and platelet derived growth factor (PDGF-BB, 50 ng/mL) were used as controls for all the assays. The results are average +/- SEM, n=3-6. Statistical analyses were performed by Mann-Whitney test, *p<0,05; **p<0,01; ***p<0,001.
   (B1) *In vivo* effect of miR-150 in wound healing of excisional wounds inflicted in C57Bl/6. The results are average +/- SEM, n=5-6. Statistical analyses were performed by t-test, p<0,05 for Scramble treatment (*); for 'No treat.' = PBS (#). (B2) Representative images at day 0; 7 and 10 of the wounds treated with miR-150-5p, Scramble and PBS (No treat.).

### Detailed Description of the Invention

The present invention refers to a process for preparing a composition comprising exosomes secreted by UCBMNCs, to compositions prepared by this process and to the use of said compositions in the medical area for therapeutical purposes, namely, to treat skin conditions.

The process for preparing an exosome composition comprising exosomes secreted by UCBMNCs, according to the present invention, comprises a step wherein said UCBMNCs are treated under specific hypoxia conditions of 0.5% O₂, 5% CO₂, during 18h in a culture medium depleted of foetal bovine serum (0% FBS), and being the secreted exosomes separated from the UCBMNCs and suspended in a suitable carrier.

The UCBMNCs comprise lymphocytes, monocytes, neutrophils, eosinophils, and/or basophils.

In one embodiment, exposure to ischemic and/or hypoxic conditions stimulates increased secretion of exosomes from UCBMNCs.

In another embodiment, exposure to ischemic and/or hypoxic conditions increased bioactivity of the exosomes secreted.

In one embodiment, the bioactivity is pro-survival bioactivity.

In another embodiment, the bioactivity is pro-proliferation bioactivity. In another embodiment, the bioactivity is pro-migration bioactivity.

In one embodiment, at least one of the exosomes exhibit a spherical morphology.

In one embodiment, the exosomes have an average particle size of 100 nm to 200 nm. In one embodiment, the exosomes have an average particle size of 120 nm to 170 nm. In one embodiment, the exosomes have an average particle size of 130 nm to 150 nm. In one embodiment, the exosomes have an average particle size of 140 nm.

In one embodiment, the exosomes have a negative zeta potential of -10mV to -50mV. In one embodiment, the exosomes have a negative zeta potential of -20 mV to -40 mV. In one embodiment, the exosomes have a negative zeta potential of -30mV.

In one embodiment, at least one of the exosomes express the markers CD9, CD34, CD81, and/or Hsc70.

In one embodiment, at least one of the exosomes contain one or more miRNA selected from a group consisting of: hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, and hsa-miR-101-3p.

In one embodiment, at least one of the exosomes contain one or more miRNA selected from a group consisting of: hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, and hsa-miR-146b-5p.

In one embodiment, at least one of the exosomes contain one or more miRNA selected from a group consisting of: hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, and hsa-miR-146a-5p.

In one embodiment, at least one of the exosomes contain one or more miRNA selected from a group consisting of: hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, and hsa-miR-342-3p.

In one embodiment, at least one of the exosomes contain one or more miRNA selected from a group consisting of: hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, and hsa-miR-26a-1-5p. In one embodiment, at least one of the exosomes contain one or more miRNA selected from a group consisting of: hsa-miR-150-5p, hsa-miR-16-5p, hsa-miR-142-3p, hsa-miR-223-3p, hsa-let-7g-5p, and hsa-miR-21-5p.

In one embodiment, at least one of the exosomes contain one or more miRNA selected from a group consisting of: hsa-miR-181a-5p, hsa-miR-451a, hsa-miR-103a-3p, hsa-miR-15a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, and hsa-miR-101-3p.

In one embodiment, at least one of the exosomes contain the miRNA hsa-miR-150-5p. In one embodiment, at least 10% of the exosomes contain the miRNA hsa-miR-150-5p. In one embodiment, at least 25% of the exosomes contain the miRNA hsa-miR-150-5p. In one embodiment, at least 50% of the exosomes contain the miRNA hsa-miR-150-5p. In one embodiment, at least 75% of the exosomes contain the miRNA hsa-miR-150-5p. In one embodiment, at least 90% of the exosomes contain the miRNA hsa-miR-150-5p.

In one embodiment, the miRNA hsa-miR-150-5p has a higher concentration in the exosomes than the concentration of any other one miRNA in the exosomes.

In one embodiment, the exosomes are enriched in one or more miRNA selected from a group consisting of: hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, and hsa-miR-101-3p, thereby increasing the concentration of the miRNA in the exosomes.

In one embodiment, the exosomes are enriched in one or more miRNA selected from a group consisting of: hsa-miR-181a-5p, hsa-miR-451a, hsa-miR-103a-3p, hsa-miR-15a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, and hsa-miR-101-3p, thereby increasing the concentration of the miRNA in the exosomes. In one embodiment, the exosomes are enriched in miRNA hsa-miR-150-5p, thereby increasing the concentration of the miRNA in the exosomes.

The exosomes may be enriched in miRNA using conventional methods known in the art including but not limited to differential centrifugation, density gradient/cushion centrifugation, size-exclusion chromatography, precipitation, immune-affinity capture on beads, and RNA Lading techniques such as techniques based on electroporation, virus infection of the secreting cells, and targeted embedding of specific RNA sequences via sequence-recognition domains fused to vesicular proteins.

In one embodiment, the composition is a liquid. In one embodiment, the composition is a cream. In one embodiment, the composition is a gel.

In one embodiment, the composition is administered in a single application. In another embodiment, the composition is administered periodically. In another embodiment, the composition is administered less often than once daily. In another embodiment, the composition is administered once daily. In another embodiment, the composition is administered more often than once daily. In another embodiment, the composition administered twice daily. In another embodiment, the composition administered three times daily.

In one embodiment, the composition is applied topically. In one embodiment, the composition is applied by subcutaneous injection. In one embodiment, the composition is applied by dermal injection. In one embodiment, the composition is applied by intraperitoneal injection. In one embodiment, the composition is applied by intravenous injection.

In one embodiment, the concentration of exosomes in the composition is 0.01 **µ**g/mL-10 **µ**g/mL. In one embodiment, the concentration of exosomes is 1 **µ**g/mL-3 **µ**g/mL. In one embodiment, the concentration of exosomes is 0.5 **µ**g/mL-1.5 **µ**g/mL. In one embodiment, the concentration of exosomes is about 1 **µ**g/mL. In one embodiment, the concentration of exosomes is 0.01 **µ**g/mL. In one embodiment, the concentration of exosomes is 0.25 **µ**g/mL. In one embodiment, the concentration of exosomes is 0.5 **µ**g/mL. In one embodiment, the concentration of exosomes is 0.75 **µ**g/mL. In one embodiment, the concentration of exosomes is 1.0 **µ**g/mL. In one embodiment, the concentration of exosomes is 1.25 **µ**g/mL. In one embodiment, the concentration of exosomes is 1.5 **µg**g/mL. In one embodiment, the concentration of exosomes is 1.75 **µ**g/mL. In one embodiment, the concentration of exosomes is 2.0 **µ**g/mL. In one embodiment, the concentration of exosomes is 2.25 **µ**g/mL. In one embodiment, the concentration of exosomes is 2.5 **µ**g/mL. In one embodiment, the concentration of exosomes is 2.75 **µ**g/mL. In one embodiment, the concentration of exosomes is 3.0 **µ**g/mL. In one embodiment, the concentration of exosomes is 5.0 **µ**g/mL. In one embodiment, the concentration of exosomes is 10 **µ**g/mL.

In one embodiment, the composition further comprises one or more miRNA selected from a group consisting of: hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, and hsa-miR-101-3p, wherein the miRNA is not contained in the exosomes.

In one embodiment, the composition further comprises one or more miRNA selected from a group consisting of: hsa-miR-181a-5p, hsa-miR-451a, hsa-miR-103a-3p, hsa-miR-15a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, and hsa-miR-101-3p, wherein the miRNA is not contained in the exosomes.

In one embodiment, the composition further comprises miRNA hsa-miR-150-5p wherein the miRNA hsa-miR-150-5p is not contained in the exosomes.

In one embodiment, wound healing is promoted by accelerating the rate of wound healing. In one embodiment, wound healing is promoted by inducing vascularization.

In one embodiment, wound healing is promoted by increasing survival of cells. In one embodiment, wound healing is promoted by increasing survival of keratinocytes. In another embodiment, wound healing is promoted by stimulating survival of endothelial cells.

In one embodiment, wound healing is promoted by increasing proliferation of cells. In one embodiment, wound healing is promoted by increasing proliferation of fibroblasts.

In one embodiment, wound healing is promoted by increasing migration of cells towards the wound. In one embodiment, wound healing is promoted by increasing migration of fibroblasts toward the wound. In another embodiment, wound healing is promoted by increasing migration of keratinocytes toward the wound.

In one embodiment, wound healing is promoted by increasing tube-formation at and/or near the site of the wound. In one embodiment, wound healing is promoted by inducing tube-formation by endothelial cells.

In one embodiment, the exosomes are effective to increase the total number of vascular tubes formed. In another embodiment, the exosomes are effective to increase the complexity of the vascular network formed. In one embodiment, the complexity of the vascular network formed is assessed by the augmented number of branching points.

In one embodiment, the keratinocytes have been exposed to ischemic conditions. In one embodiment the keratinocytes have been exposed to hypoxic conditions. In one embodiment, the fibroblasts have been exposed to ischemic conditions. In one embodiment the fibroblasts have been exposed to hypoxic conditions. In one embodiment, the endothelial cells have been exposed to ischemic conditions. In one embodiment the endothelial cells have been exposed to hypoxic conditions.

In one embodiment, exosomes exposed to ischemic conditions are more effective in inducing proliferation of fibroblasts than exosomes not exposed to ischemic conditions.

In one embodiment, exosomes exposed to ischemic conditions are more effective in inducing proliferation of fibroblasts at a concentration of 1 **µ**g/mL than exosomes not exposed to ischemic conditions at a concentration of 1 **µ**g/mL. In one embodiment, exosomes exposed to ischemic conditions are more effective in inducing proliferation of fibroblasts at a concentration of 2 **µ**g/mL than exosomes not exposed to ischemic conditions at a concentration of 2 **µ**g/mL.

In another embodiment, exosomes exposed to hypoxic conditions are more effective in inducing proliferation of fibroblasts. In one embodiment, exosomes exposed to hypoxic conditions are more effective in inducing proliferation of fibroblasts at a concentration of 1 **µ**g/mL. In one embodiment, exosomes exposed to hypoxic conditions are more effective in inducing proliferation of fibroblasts at a concentration of 2 **µ**g/mL.

In one embodiment, wound healing is promoted by the miRNA hsa-miR-150-5p.

In another embodiment, the miRNA hsa-miR-150-5p increases migration of keratinocytes towards the wound thereby promoting wound healing. In another embodiment, the miRNA hsa-miR-150-5p increases survival of fibroblast survival thereby promoting wound healing.

In one embodiment, wound healing is promoted at a site local to the site of contact of the exosomes. In another embodiment, wound healing is promoted at a site distant from the site of contact of the exosomes.

In another embodiment, the wound is a chronic wound.

In one embodiment, the wound is a pressure ulcer. In another embodiment, the wound is a venous ulcer. In another embodiment, the wound is a post-surgical ulcer. In another embodiment, the wound is a traumatic ulcer. In another embodiment, the wound is a lower extremity wound. In another embodiment, the wound is an arterial ulcer.

In one embodiment, the wound is a mouth ulcer.

In one embodiment, the wound is an eye wound or a corneal ulcer.

In one embodiment, the patient is afflicted with diabetes. In one embodiment, the patient is not afflicted with diabetes.

In another embodiment, the patient is afflicted with type I diabetes. In another embodiment, the patient is afflicted with type II diabetes.

In one embodiment, the wound is a diabetic ulcer. In one embodiment, the diabetic ulcer is a diabetic foot ulcer.

In one embodiment, the wound is an open wound. In one embodiment, the open wound is an incision. In one embodiment, the open wound is a laceration. In one embodiment, the open wound is a tear. In one embodiment, the open wound is an abrasion. In one embodiment, the open wound is an avulsion. In one embodiment, the open wound is a puncture.

In one embodiment, the wound is an excisional wound. In another embodiment, the wound is an infectious wound. In another embodiment, the wound is an ischemic wound. In another embodiment, the wound is a radiation-poisoning wound. In another embodiment, the wound is a surgical wound.

In one embodiment, the wound is a burn. In one embodiment, the burn is a thermal burn. In one embodiment, the burn is a chemical burn. In one embodiment, the burn is a radiation burn.

In one embodiment, the wound relates to a skin condition or disease comprising a wound such as acne, psoriasis, rosacea, dermatitis, eczema, impetigo, intertrigo, or folliculitis.

In one embodiment, the accelerated rate of wound healing is significant by day 2. In another embodiment, the accelerated rate of wound healing is significant by day 3. In another embodiment, the accelerated rate of wound healing is significant by day 10.

In one embodiment, wound healing is assessed by wound closure. In one embodiment, wound closure is improved by at least 10%. In another embodiment, wound closure is improved by at least 20%. In another embodiment, wound closure is improved by at least 30%. In another embodiment, wound closure is improved by at least 50%. In another embodiment, wound closure is improved by at least 80%. In another embodiment, wound closure is improved by more than 100%.

In one embodiment, the time taken for complete wound closure is reduced by at least 10%. In another embodiment, the time taken for complete wound closure is reduced by at least 20%. In another embodiment, the time taken for complete wound closure is reduced by at least 30%. In another embodiment, the time taken for complete wound closure is reduced by at least 50%. In another embodiment, the time taken for complete wound closure is reduced by at least 80%. In another embodiment, the time taken for complete wound closure is reduced by more than 100%.

The present invention provides a process of making a composition comprising exosomes secreted by umbilical cord blood mononuclear cells (UCBMNCs) as claimed in claim 1 comprising step a) separating the exosomes from the UCBMNCs and b) suspending the exosomes in a pharmaceutically acceptable carrier.

In one embodiment, the pharmaceutically acceptable carrier is phosphate buffered saline (PBS). In one embodiment, the pharmaceutically acceptable carrier is a liquid suspension. In one embodiment, the pharmaceutically acceptable carrier is a polymer. In one embodiment, the pharmaceutically acceptable carrier is a hydrogel. In one embodiment, the pharmaceutically acceptable carrier is protein matrix. In one embodiment, the pharmaceutically acceptable carrier is a liposomal suspension. In one embodiment, the pharmaceutically acceptable carrier is a foam.

In one embodiment, the exosomes are separated from the UCBMNCs by centrifugation. In one embodiment, the exosomes are separated from the UCBMNCs by differential centrifugation.

The most commonly used technique to purify exosomes is differential ultracentrifugation, but other techniques may also be used for the subject invention including but not limited to ultracentrifugation in a density gradient, high-pressure liquid chromatography-gel exclusion chromatography (HPLC-GEC), solvent precipitation, ultrafiltration, immunoaffinity capture (IAC), and field flow fractionation (FFF).

Solvent precipitation is a technique that successfully separates exosomes at relatively slow spin speeds without an ultracentrifuge. Three examples of commercial kits using solvent precipitation are Exosome Isolation kit (Life Technologies), ExoQuick (System Bioscience), and Exo-spin^{™} (Cell Guidance System).

IAC relies on the binding of an antibody to its specific antigen. As the knowledge of the subcomponents of exosomes grows, more individual antigen targets (typically proteins) are identified that can be used to specifically identify exosomes.

FFF is a technique where particles are separated by their position in a laminar velocity gradient. In asymmetrical flow field flow fractionation, a crossflow perpendicular to the down-channel flow drives particles toward the membrane that bounds the channel. Diffusion causes particles to migrate away from the membrane, opposing the crossflow.

In one embodiment, the process further comprises a step of enriching the exosomes in one or more miRNA prior to step b), wherein the miRNA is selected from a group consisting of: has-miR-150-5p, has-miR-16-5p, has-miR-142-3p, has-miR-223-3p, has-let-7g-5p, has-miR-21-5p, has-let-7f-5p, has-miR-19b-3p, has-let-7a-5p, has-miR-26a-1-5p, has-miR-20a-5p, has-miR-181a-5p, has-miR-451a, has-miR-23a-3p, has-miR-342-3p, has-miR-191-5p, has-miR-103a-3p, has-miR-15a-5p, has-miR-142-5p, has-miR-146a-5p, has-miR-19a-3p, has-miR-15b-5p, has-miR-26b-5p, has-miR-30d-5p, has-miR-146b-5p, has-miR-106b-5p, has-miR-29a-3p, has-miR-17-5p, has-miR-29b-3p, and has-miR-101-3p.

In one embodiment, the process further comprises a step of enriching the exosomes in one or more miRNA prior to step b), wherein the miRNA is selected from a group consisting of: has-miR-181a-5p, has-miR-451a, has-miR-103a-3p, has-miR-15a-5p, has-miR-19a-3p, has-miR-15b-5p, has-miR-26b-5p, has-miR-30d-5p, has-miR-146b-5p, has-miR-106b-5p, has-miR-29a-3p, has-miR-17-5p, has-miR-29b-3p, and has-miR-101-3p.

In one embodiment, the process further comprises a step of enriching the exosomes in miRNA has-miR-150-5p prior to step b).

In one embodiment, the composition further comprises one or more miRNA not contained in the exosomes and the process further comprises the steps of mixing the miRNA with a transfection agent and suspending the mixture in the pharmaceutically acceptable carrier, wherein the miRNA is selected from a group consisting of has-miR-150-5p, has-miR-16-5p, has-miR-142-3p, has-miR-223-3p, has-let-7g-5p, has-miR-21-5p, has-let-7f-5p, has-miR-19b-3p, has-let-7a-5p, has-miR-26a-1-5p, has-miR-20a-5p, has-miR-181a-5p, has-miR-451a, has-miR-23a-3p, has-miR-342-3p, has-miR-191-5p, has-miR-103a-3p, has-miR-15a-5p, has-miR-142-5p, has-miR-146a-5p, has-miR-19a-3p, has-miR-15b-5p, has-miR-26b-5p, has-miR-30d-5p, has-miR-146b-5p, has-miR-106b-5p, has-miR-29a-3p, has-miR-17-5p, has-miR-29b-3p, and has-miR-101-3p.

In one embodiment, the miRNA is selected from a group consisting of has-miR-181a-5p, has-miR-451a, has-miR-103a-3p, has-miR-15a-5p, has-miR-19a-3p, has-miR-15b-5p, has-miR-26b-5p, has-miR-30d-5p, has-miR-146b-5p, has-miR-106b-5p, has-miR-29a-3p, has-miR-17-5p, has-miR-29b-3p, and has-miR-101-3p.

In one embodiment, the miRNA is has-miR-150-5p.

The invention also provides a composition comprising exosomes secreted by umbilical cord blood mononuclear cells (UCBMNCs) prepared by the process of the invention.

The invention also provides a wound care product comprising exosome composition of the invention.

In one embodiment, the amount of miRNA has-miR-150-5p applied is between 100 pmol to 1 nmol. In one embodiment, the amount of miRNA has-miR-150-5p applied is between 100 pmol to 800 pmol. In one embodiment, the amount of miRNA has-miR-150-5p applied is between 300 pmol to 500 pmol. In one embodiment, the amount of miRNA has-miR-150-5p applied is 400 pmol.

Disclosed herein but not part of the invention is a composition comprising miRNA selected from a group consisting of: has-miR-150-5p, has-miR-181a-5p, has-miR-451a, has-miR-103a-3p, has-miR-15a-5p, has-miR-19a-3p, has-miR-15b-5p, has-miR-26b-5p, has-miR-30d-5p, has-miR-146b-5p, has-miR-106b-5p, has-miR-29a-3p, has-miR-17-5p, has-miR-29b-3p, and has-miR-101-3p for promoting wound healing in a patient in need thereof. In one aspect, one of the miRNA is has-miR-150-5p.

In one aspect, the composition further comprises a pharmaceutically acceptable carrier.

Disclosed herein but not part of the invention is a process of making a composition comprising one or more miRNA and a pharmaceutically acceptable carrier comprising step a) mixing the miRNA with a transfection agent, and b) suspending the mixture in a pharmaceutically acceptable carrier, wherein the miRNA is selected from a group consisting of: has-miR-150-5p, has-miR-181a-5p, has-miR-451a, has-miR-103a-3p, has-miR-15a-5p, has-miR-19a-3p, has-miR-15b-5p, has-miR-26b-5p, has-miR-30d-5p, has-miR-146b-5p, has-miR-106b-5p, has-miR-29a-3p, has-miR-17-5p, has-miR-29b-3p, and has-miR-101-3p.

In one aspect, one of the miRNA is has-miR-150-5p.

Disclosed herein but not part of the invention is a composition comprising miRNA and a pharmaceutically acceptable carrier prepared by the processes described herein.

The compositions of the invention can be used to provide a wound care product such as, a dressing or a pharmaceutical preparation. Such dressing or pharmaceutical preparation can be a graft material, patch, pad, plaster, film, tape, adhesive, gel, foam, cream, salve, balm, embrocation, ointment, poultice, unction, emollient, liniment, potion, unguent, lotion, spray, suspension, powder, syringe, nebuliser or aerosol container.

The wound care product is **preferably:**
a) a graft material comprising at least one fibroblast layer,
b) a gel or graft material comprising a fibrin matrix and fibroblasts,
c) a graft material comprising a matrix of at least one structural protein,
d) a graft material comprising a matrix of cross-linked collagen and glycosaminoglycan,
e) a gel, patch, pad, plaster, film or adhesive comprising a matrix of hydrophilic polymers dispersed in water,
f) a polyurethane film and/or a foam,
g) a pharmaceutical preparation comprising extracellular matrix protein, alginate and water, or
h) an aerosol container comprising an aerosol comprising said exosomes.

Preferably, the wound care product further comprises an antiseptic or antibacterial agent.

The composition of the invention is used in the manufacturing of a medicament for promoting wound healing in a patient in need thereof.

### Terms

Unless otherwise defined, all technical and scientific terms used herein have the same meaning as commonly understood by a person of ordinary skill in the art to which this invention belongs.

As used herein, and unless stated otherwise or required otherwise by context, each of the following terms shall have the definition set forth below.

The term "wound" as used herein refers to an injury to any living tissue. A wound may be caused by an act, by an infectious disease, or by an underlying condition. A wound may be chronic or acute. Examples of wound include but is not limited to cuts, ulcers, diabetic ulcers, ischemic tissue damage, ischemic heart damage, etc.

This invention will be better understood by reference to the Experimental Details which follow, but those skilled in the art will readily appreciate that the specific experiments detailed are only illustrative of the invention as defined in the claims which follow thereafter.

### Experimental Details

### Example 1. Ischemic Preconditioning Stimulates Umbilical Cord Blood Mononuclear Cells (UCBMNCs) to Secrete Exosomes and Increases Their Bioactivity towards Skin Cells

The total fraction of UCBMNCs was isolated using Lymphoprep^{™} and the cells were characterized using a haematological analyser. After cryopreservation, the population is enriched in lymphocytes and monocytes (MNC), while a significant fraction of neutrophils is lost (Figure 1A and 1B). The pool used for secretion of exosomes is mainly composed of lymphocytes and monocytes.

The experiments demonstrated that ischemic pre-conditioning (0.5% O₂, 5% CO₂, 0% FBS, 18h) stimulates UCBMNCs to secrete bioactive exosomes which are internalized with specific kinetics by different types of skin cells (fibroblasts, keratinocytes and endothelial cells). The experiments also demonstrated that these exosomes (UCBMNCs-Exo) exert a potent pro-regenerative effect *in vitro,* which involves increasing the survival and proliferation of skin cells (fibroblasts, keratinocytes, and endothelial cells), as well as stimulating migration of fibroblasts and keratinocytes and tube formation by endothelial cells. Furthermore, the experiments demonstrated that topical administration of UCBMNCs-Exo twice a day significantly accelerates the healing of excisional wounds in normal and in Type 1 and 2 diabetic animal models.

### Example 1.1: Characterization of Exosomes

### Experimental Procedure:

Exosomes were purified by differential centrifugation as described in Thery C. et al. (2006), the entire content of which is hereby incorporated.

Briefly, the supernatant was subjected to sequential centrifugation steps of 300g (10 minutes), 2000g (20 minutes), and two times at 10,000g (30 minutes), for removal of cells, cell debris, and microvesicles, respectively. After a 2h centrifugation at 100000g in a SW41Ti or SW32Ti swinging bucket rotors (Beckman) the pellet was washed with 7 mL of PBS and centrifuged at 100000g for another 2h. The pellet containing the exosomes was re-suspended in 250uL of PBS. The exosomes were quantified based in their protein content using a DC Protein assay Kit from BioRad^{™}.

The exosomes secreted by UCBMNC were initially characterized using transmission electron microscopy (TEM) and dynamic light scattering (DLS). The DLS analyses were performed in a Zeta PALS Zeta Potential Analyzer, containing a ZetaPlus Particle Sizing Software, v. 2.27 (Brookhaven Instruments Corporation). For particle size, exosomes (50 µL) were diluted in PBS (200 µL) and five measurements per sample were acquired at 25°C for n=17. For the zeta potential, exosomes (50 µL) were diluted 25 times with biological molecular grade water filtered through a 0.2 µm pore (final PBS concentration of 5 mM). Each sample was measured 5 times and the results presented are the arithmetic means of n=4. The TEM analyses were performed in a Jeol JEM 1400 transmission electron microscope (Tokyo). The samples were mounted on 300 mesh formvar copper grids, stained with uranyl acetate 1%. Images were digitally recorded using a Gatan SC 1000 ORIUS CCD camera (Warrendale, PA, USA), and photomontages were performed using Adobe Photoshop CS software (Adobe Systems, San Jose, CA), at the HEMS / IBMC - Institute for Molecular and Cell Biology (IBMC) of the University of Porto, Portugal.

### Results:

Our results indicated that the exosomes had an average size of 140 nm (Figure 2A), a negative zeta potential (-30mV) (Figure 2B), and a spherical morphology (Figure 2C.1 and 2C.2). In addition, these exosomes expressed the markers CD9, CD81 and HSC70 as analysed by FACS (Figure 2D) .

There was no significant difference between the biophysical characteristics of exosomes secreted in hypoxia and that of those secreted in normoxia (control conditions). However, the data suggested that hypoxia stimulated increased secretion of exosomes by UCBMNCs, as observed from the increased protein content (Figure 2E), which are normalized to the same number of secreting cells and re-suspended in equivalent final volumes.

### Example 1.2: Exosome Uptake Kinetics by Skin Cells

### 1.2.1 Percentage of Skin Cells

### Experimental Procedure:

Skin cells uptake exosomes derived from UCBMNC, however the uptake kinetics and magnitude are dependent on the cell type. To demonstrate the differences in the exosome uptake kinetics by skin cells, exosomes were initially labelled with a fluorescent dye (NBD-DPPE and Syto green). Briefly, NBD-DPPE (16 µM in ethanol) or Syto Green (10 µM in DMSO) dyes were added to an exosome suspension (100 µL; protein concentration ranging from 60 to 70 µg/mL) (DMSO and EtOH <1%) and incubated at 37°C for 30 min. After blocking the reaction with bovine serum albumin (BSA, 1%, w/v) the exosomes were washed with PBS (6 mL) and the free dye was removed by ultracentrifugation at 100,000g for 1 h. The exosomes were then re-suspended in PBS (100 µL). This procedure was always performed with fresh exosomes. The labelled exosomes were used immediately in the internalization experiments.

We evaluated the percentage of skin cells able to uptake UCBMNC-derived exosomes after 4 h of incubation, by flow cytometry. HUVECs (80,000 cells/well), HaCaT cells (160,000 cells/well) and fibroblasts (50,000 cells/well) were seeded in a 24 well-plate and left to grow for 18-24 h. The confluent cells were incubated with NBD-DPPE labelled exosomes for 4 h in cell culture media without foetal bovine serum (FBS), at final concentrations of 1, 2 and 3 µg/mL. Afterwards, cells were washed once with PBS, detached with trypsin (HUVEC and NDHF) or tripLE^{™} Express (HaCaT), washed twice in PBS and analysed by flow cytometry on a FACS-Calibur instrument integrated with Cell-Quest software (BD Biosciences).

### Results:

Exosomes are not cytotoxic to endothelial cells, for concentrations up to 10 **µ**g/mL (Figure 3A).

Our results also showed that for concentrations of exosomes between 1 and 3 µg/mL, higher internalization levels were observed for higher concentrations of exosomes (Figure 3B). In addition, both keratinocytes and fibroblasts were more permissive to exosome internalization than endothelial cells.

### 1.2.2: Effect of Incubation Time

### Experimental Procedure:

Next, we evaluated the influence of incubation time and concentration of exosomes in the uptake kinetics of exosomes by skin cells using confocal microscopy (Figure 3C). The snapshots (Figure 3C) were acquired with a Zeiss LSM 710 confocal scanning equipment, using the excitation wavelengths of, 405 and 488 and a Plan-Apochromat 40×/1.4 oil immersion objective. Kinetic profile was also obtained by analysing the cytoplasm intensity at different time points for 24h, using high-content microscopy (In Cell Analyser, GE Healthcare). The microscope is equipped with heat incubator which was set at 37 °C and a CO2 supply set to 5%. For live cell microscopy experiments, HUVEC cells were grown for 24 h in an *IBIDI^{™}* µ-Slide *angiogenesis plate.* Syto^{®} RNASelect^{™} labelled exosomes (1-20 µg/ml) were added to confluent cells in exo-depleted medium and incubated for different time points. Cells were washed two times with PBS and kept in FBS containing medium during the course of the experiments.

### Results:

Our results showed that in the three types of cells tested, exosomes were located in the overall cytoplasm. Accumulation of exosomes in the cell cytoplasm peaked at approximately 16 h after contact (Figure 3D).

### 1.2.3: Functional Effects of Exosomes

### Experimental Procedure:

To determine functional effects of exosomes on skin cells, we evaluated whether exosome internalization could induce (i) cell survival under ischemic conditions, (ii) cell proliferation, (iii) cell migration and (iv)endothelial tube formation (Figures 4 and 5). For cell survival experiments, HUVECs (1×10⁴) were cultured in EGM-2 (Lonza), with 2% FBS, NDHFs (5×10³) and HaCaT (2×10⁴) were cultured in DMEM (Invitrogen), with 0.5% Penstrep and 10% FBS, in 96 plates. After 20 h, cells were washed with PBS and incubated with exo-depleted medium alone (100 µL) or with exosomes (100 µL, containing 1 µg/mL, 2 µg/mL, 3 µg/mL or 5 µg/mL) for 48 h. Then cells were incubated in ischemia conditions for 48 h (HUVECs in EGM-2 without VEGF and FBS; and HaCaT in DMEM without FBS at 37°C, 5% CO₂, 0.1% O2) or 72h (fibroblasts in DMEM without FBS at 37°C, 5% CO₂, 0.1% O2). At the end the cells were monitored by high-content microscopy and cell viability was quantified by hoescht/propidium iodide (PI) staining. Cell survival was calculated according to the following equation: (Cell survival_{treatment group} - Cell survival_{control group}) / Cell survival_{control group}.

The following procedure was adopted to demonstrate the proliferation inductive properties of exosomes. HUVECs (2.5×10³) were cultured in EGM-2 (Lonza), with 2% FBS, fibroblasts (5×10³) and HaCaT (2.5×10³) were cultured in DMEM (Invitrogen), with 0.5% Penstrep and 10% FBS, in 96 plates. After 20 h, cells were stained with Hoechst (33342, Life Technologies) for 15 min, washed with PBS and incubated in exo-depleted medium alone (100 µL) or with exosomes (1 µg/mL, 2 µg/mL or 3 µg/mL) for 24 h. Then the medium was changed, and cells were incubated (37°C, 5% CO2, 0.1% O2) in exo-depleted medium (200 µL) for 72 h. Cells were stained with Hoechst and photographed (IN Cell, GE Healthcare) every 24 h and cell number was calculated by counting cell nuclei. Proliferation ratio was obtained by dividing the cell number at 72h after treatment by the number of cells at the time of the incubation with exosomes (Time zero - T0).

Fibroblasts (1x10⁴) and HaCaT cells (2x10⁴) were cultured in DMEM (Invitrogen), with 0.5% Pen Strep and 10% FBS, in 96 plates. After 20 h, a vertical scratch was made with a 200 µl pipette tip in the middle of the well and cells were washed with PBS. Then, exo-depleted medium alone (100 µL) or supplemented with exosomes (1 µg/mL, 2 µg/mL or 3 µg/mL) was added to the cells and images of the wells (time zero T0) were taken in In Cell Analyzer (GE Healthcare). After 4 h, further exo-depleted medium was added (100 µL) and cells incubated for 18 h. Afterwards, cell media was changed to exo-depleted DMEM with 0.5% FBS, and cells were incubated at 37°C, 5% CO₂, 20% O2 for 48 h. Pictures were taken 48 h after scratch and wound area measured in AxioVision software 4.6.3 (Carl Zeiss Imaging Solutions GmbH).

To determine the effect of exosomes on tube formation in endothelial cells, HUVECs (5x10⁴/well, passage 3 - 5) were cultured in EGM-2 (Lonza) in 24 well plate over-night. After 20 h, cells were incubated in 200 µL of exo-depleted medium alone and with exosomes (1 µg/mL, 2 µg/mL, 3 µg/mL and 5 µg/mL). After 48 h cells were trypsinized and seeded in EBM-2 on top of the polymerized Matrigel (BD Biosciences, 10 µL per well, 30 minutes at 37°C) in a 15-well ibidi plate. Twelve hours after cell seeding, cells were photographed by phase contrast microscopy and tube formation was evaluated and quantified using WimTube software (Wimasis).

### Results:

Our results showed that exosomes isolated from UCBMNCs cultured in hypoxic conditions (Exo_Hyp), but not in normoxic conditions (Exo_Nor), were very efficient in inducing endothelial cell survival cultured in ischemic conditions for 48 h (Figure 4A.1-4).

According to our results, the survival peaked for concentrations of exosomes of 2 µg/mL. Interestingly, although both Exo_Hyp and Exo_Nor induce keratinocyte survival cultured in vitro ischemic conditions they had no effect in the survival of fibroblasts. Therefore, our results indicate that the pro-survival of exosomes is cell and concentration dependent.

Our results showed that exosomes induced the proliferation of fibroblasts at concentrations of 1 and 2 **µ**g/mL for Exo_Hyp and 2 and 3 **µ**g/mL for Exo_Nor (Figure 4B.1-4). Interestingly, exosomes had no significant effect in the proliferation of endothelial cells and keratinocytes in vitro conditions, showing one more type that exosome-induced properties are cell and concentration dependent.

Our results showed that exosomes, particularly Exo_Hyp, significantly increased mobility of fibroblast and keratinocyte cells, indicating that these exosomes also exhibit pro-migratory bioactivity (Figure 5A1-3).

Our results showed that UCBMNCs-Exo significantly increased the total number of vascular tubes and the complexity of the vascular network formed, as observed from the augmented number of branching points (Figure 5B).

These results demonstrated the effect of UCBMNCs-Exo on keratinocytes, endothelial and fibroblasts cells individually and in in vitro conditions. Keratinocytes, endothelial and fibroblast cells are the main cell types involved in wound healing indicating a role of UCBMNCs-Exo on wound healing. Since, in vitro studies fail to recapitulate the in vivo conditions, the effect of UCBMNCs-Exo on wound healing in vivo studies were performed (explained in Example 3). In the Example 3, we demonstrate the effect of UCBMNCs-Exo on wound healing.

### Example 2. Characterization of the RNA Content of UCBMNCs-Exo

### Experimental Procedure:

The RNA content of UCBMNCs-Exo was characterized by RNA deep-sequencing. UCBMNCs-Exo secreted in hypoxia and normoxia from two different donors were used. The following methodology was used.

Total RNA was extracted from the exosomes pellet with the miRCURY RNA Isolation Kit - Cell & Plant (EXIQON) using the Lysate Preparation from Cultured Animal Cells protocol. The amount and quality of total RNA and small RNA were assessed using the RNA 6000 Pico kit and the Small RNA kit in the Agilent 2100 Bioanalyzer (Agilent Technologies), respectively. Samples proceeded for cDNA library preparation using both Whole Transcriptome (WT) and Small RNA libraries protocols described in Ion Total RNA-Seq Kit v2 User Guide (Life Technologies).

All procedures were carried out according to manufacturer's instructions, except the RNA fragmentation step that was skipped in the preparation of the whole transcriptome libraries since the starting material was already of the required length.

Briefly, 2.1-4.5 ng of small RNA and 20 ng of total RNA were used to construct the small and WT libraries, respectively. RNA adaptors were ligated at 16°C for 16 h to small and at 30°C for 1 h to WT libraries. The first and second cDNA strands were synthesized, and purified cDNA was then amplified with specific barcoded primers by PCR and the resulting fragments selected for the correct size with magnetic beads. The cDNA libraries were quantified, and their quality assessed with the Agilent High Sensitivity DNA Kit for WT RNA libraries and the Agilent DNA 1000 kit for small libraries in the 2100 Bioanalyzer (Agilent Technologies). Two pools of barcoded libraries (WT and small RNA) were prepared and clonally amplified by emulsion PCR using the Ion PI Template OT2 200 kit v2 and the Ion OneTouch 2 System (Life Technologies), and the positive Ion Sphere Particles enriched in the Ion OneTouch ES machine (Life Technologies). Finally, the positive spheres were loaded into a single Ion PI chip v2 and sequenced in the Ion Proton System (Life Technologies) at Genoinseq (Cantanhede, Portugal). All procedures were carried out according to manufacturer's instructions. Ion Proton adapter sequences and low-quality bases were trimmed using the Torrent Suite software (Life Technologies).

Sequence reads were then filtered by length, where reads with less than 15 bp were removed from the small library and 18 bp for the large library. Next, rRNA, tRNA, miRNA, mRNA and ncRNA were identified by aligning the reads from each sample to their specific libraries (rRNA: 5S - NR_023379.1, 5.8S - NR_003285.2, 18S - NR_003286.2, 28S - NR_003287.2; tRNA: library of tRNA extracted from UCSC; mir: mirbase; mRNA: CDS library from Ensembl; ncRNA library from Ensembl and ENCODE) using TMAP version 4.1.

Next, we verified the expression of the 15 miRNAs by qRT-PCR in exosomes collected from 10 individuals (Figure. 6B1). After isolation of total RNA of exosomes secreted in hypoxia and normoxia by mononuclear cells of 10 different donors, all the miRNAs in the sample were polyadenylated and a cDNA library was constructed using the NCode^{™} miRNA first-strand cDNA synthesis and qRT-PCR Kit (Invitrogen^{™}, Life Technologies, Carlsbad, California, USA) and the expression of the 15 most expressed microRNAs, as determined by the RNASeq data, was confirmed by qRT-PCR using the Fluidigm dynamic array in the BioMark^{™} system (Fluidigm corporation, South San Francisco, CA, USA), following the manufacturer's instructions. Specific primers for each microRNA were designed accordingly to NCode kit instructions and U6 snRNA was used as endogenous control (Wang Y, et al, 2015).

### Results:

Our results showed that both Exo-Hyp and Exo_Nor have a large pool of miRNAs (Figure 6A). In terms of total reads, the 30 most representative miRNAs for both exosomes were: hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, and hsa-miR-101-3p.

The accession number and mature sequences of the 30 most representative miRNAs are summarized in Table I below.

**Table I - Mature microRNA in UCBMNCs exosomes, miRBASE accession number and sequence**

| **#** | **Name** | **Accession number** | **Mature sequence** |
|---|---|---|---|
| 1 | hsa-mir-150-5p | MIMAT0000451 | UCUCCCAACCCUUGUACCAGUG |
| 2 | hsa-mir-16-5p | MIMAT0000069 | UAGCAGCACGUAAAUAUUGGCG |
| 3 | hsa-mir-142-3p | MIMAT0000434 | UGUAGUGUUUCCUACUUUAUGGA |
| 4 | hsa-mir-223-3p | MIMAT0000280 | UGUCAGUUUGUCAAAUACCCCA |
| 5 | hsa-let-7g-5p | MIMAT0000414 | UGAGGUAGUAGUUUGUACAGUU |
| 6 | hsa-mir-21-5p | MIMAT0000076 | UAGCUUAUCAGACUGAUGUUGA |
| 7 | hsa-let-7f-5p | MIMAT0000067 | UGAGGUAGUAGAUUGUAUAGUU |
| 8 | hsa-mir-19b-3p | MIMAT0000074 | UGUGCAAAUCCAUGCAAAACUGA |
| 9 | hsa-let-7a-5p | MIMAT0000062 | UGAGGUAGUAGGUUGUAUAGUU |
| 10 | hsa-mir-26a-1-5p | MIMAT0000082 | UUCAAGUAAUCCAGGAUAGGCU |
| 11 | hsa-mir-20a-5p | MIMAT0000075 | UAAAGUGCUUAUAGUGCAGGUAG |
| 12 | hsa-miR-181a-5p | MIMAT0000256 | AACAUUCAACGCUGUCGGUGAGU |
| 13 | hsa-miR-451a | MIMAT0001631 | AAACCGUUACCAUUACUGAGUU |
| 14 | hsa-miR-23a-3p | MIMAT0000078 | AUCACAUUGCCAGGGAUUUCC |
| 15 | hsa-miR-342-3p | MIMAT0000753 | UCUCACACAGAAAUCGCACCCGU |
| 16 | hsa-miR-191-5p | MIMAT0000440 | CAACGGAAUCCCAAAAGCAGCUG |
| 17 | hsa-miR-103a-3p | MIMAT0000101 | AGCAGCAUUGUACAGGGCUAUGA |
| 18 | hsa-miR-15a-5p | MIMAT0000068 | UAGCAGCACAUAAUGGUUUGUG |
| 19 | hsa-miR-142-5p | MIMAT0000433 | CAUAAAGUAGAAAGCACUACU |
| 20 | hsa-miR-146a-5p | MIMAT0000449 | UGAGAACUGAAUUCCAUGGGUU |
| 21 | hsa-miR-19a-3p | MIMAT0000073 | UGUGCAAAUCUAUGCAAAACUGA |
| 22 | hsa-miR-15b-5p | MIMAT0000417 | UAGCAGCACAUCAUGGUUUACA |
| 23 | hsa-miR-26b-5p | MIMAT0000083 | UUCAAGUAAUUCAGGAUAGGU |
| 24 | hsa-miR-30d-5p | MIMAT0000245 | UGUAAACAUCCCCGACUGGAAG |
| 25 | hsa-miR-146b-5p | MIMAT0002809 | UGAGAACUGAAUUCCAUAGGCU |
| 26 | hsa-miR-106b-5p | MIMAT0000680 | UAAAGUGCUGACAGUGCAGAU |
| 27 | hsa-miR-29a-3p | MIMAT0000086 | UAGCACCAUCUGAAAUCGGUUA |
| 28 | hsa-miR-17-5p | MIMAT0000070 | CAAAGUGCUUACAGUGCAGGUAG |
| 29 | hsa-miR-29b-3p | MIMAT0000100 | UAGCACCAUUUGAAAUCAGUGUU |
| 30 | hsa-miR-101-3p | MIMAT0000099 | UACAGUACUGUGAUAACUGAA |

The sequences of the forward primers used for qRT-PCR for the mature miRNAs and RNU6 are summarized in Table II below.

**Table II - Mature microRNA and RNU6 accession numbers (miRBase and GenBank) and sequences of the forward primers used for qRT-PCR**

| **#** | **Name** | **Accession number** | **Forward Primer** |
|---|---|---|---|
| 1 | hsa-mir-150-5p | MIMAT0000451 | TCTCCCAACCCTTGTACC |
| 2 | hsa-mir-16-5p | MIMAT0000069 | TAGCAGCACGTAAATATTG |
| 3 | hsa-mir-142-3p | MIMAT0000434 | TGTAGTGTTTCCTACTTTATGGA |
| 4 | hsa-mir-223-3p | MIMAT0000280 | TGTCAGTTTGTCAAATACCC |
| 5 | hsa-let-7g-5p | MIMAT0000414 | TGAGGTAGTAGTTTGTACAGTT |
| 6 | hsa-mir-21-5p | MIMAT0000076 | TAGCTTATCAGACTGATGTTGA |
| 7 | hsa-let-7f-5p | MIMAT0000067 | TGAGGTAGTAGATTGTATAGTT |
| 8 | hsa-mir-19b-3p | MIMAT0000074 | TGTGCAAATCCATGCAAAA |
| 9 | hsa-let-7a-5p | MIMAT0000062 | TGAGGTAGTAGGTTGTATAGTT |
| 10 | hsa-mir-26a-1-5p | MIMAT0000082 | TTCAAGTAATCCAGGATAGGCT |
| 11 | hsa-mir-20a-5p | MIMAT0000075 | TAAAGTGCTTATAGTGCAGGTAG |
| 12 | hsa-miR-181a-5p | MIMAT0000256 | AACATTCAACGCTGTCGGG |
| 13 | hsa-miR-451a | MIMAT0001631 | AAACCGTTACCATTACTGAGTT |
| 14 | hsa-miR-23a-3p | MIMAT0000078 | ATCACATTGCCAGGGATT |
| 15 | hsa-miR-342-3p | MIMAT0000753 | TCTCACACAGAAATCGCAC |
| Ctr | RNU6 | NR_004394 | ACACGCAAATTCGTGAAG |

Our analysis also confirmed hsa-miR-150-5p as the most expressed with, roughly, more than 4 fold the expression of the next most expressed microRNA, hsa-miR-342a-3p (Figure 6B).

A previous report (Gray et al., 2015) has identified 7 miRNAs that were differentially expressed in exosomes collected from cardiac progenitor cells cultured in normoxia and hypoxia: miR-292, miR-210, miR-103, miR-17, miR-199a, miR-20a and miR-15b. It is important to note that most of the miRNAs identified in this patent (with the exception of miR-20a and miR-15b) are new and not previously disclosed.

### Example 3. UCBMNCs-Exo Accelerate Wound Healing In Vivo

### Experimental Procedure:

In Type 1 model, diabetes mellitus was induced by intraperitoneal injection of streptozotocin (Sigma-Aldrich) in male C57BL/6 mice (10-12 week-old), purchased from Charles River (Wilmington, MA, USA).

For a Type 2 diabetes model, db/db genetically modified mice were acquired from Charles River (Wilmington, MA, USA).

After confirming that glucose levels were greater than 300 mg/dL (six to eight weeks following diabetes induction in the Type 1 model), the animals were anesthetized, the dorsolumbar skin shaved and disinfected, and two 6 mm-diameter dorsal full-thickness excisional wounds were created with a sterile biopsy punch in each animal.

The wounds were then covered with PBS (10 µL; two times per day), Exo_Hyp(10 µL of an exosome suspension at 2µg/mL; two times per day; therefore, 0.4 µg per wound for the a 10 days overall treatment) or PDGF-BB (100 µg/mL, Peprotech; 4 µg/cm²; once a day).

After surgery, the animals were maintained in individual cages with food and water ad libitum and in a temperature and humidity-controlled environment. The wounds were measured everyday over 10-15 days. At the end of the study, animals were sacrificed by cervical dislocation and 10 mm-diameter skin biopsies were collected for further analysis.

### Results:

Our results showed that exosome treatment significantly accelerated the rate of healing of Type 1 diabetic excisional wounds (Figure 7A1 and A2). Acceleration of wound healing (smaller wound size) was observed from as early as day 2, which was statistically significant in Donor 2. After 10 days, a consistent 55% improvement of wound closure upon application of UCBMNCs-Exo was observed (size of the treated wound is half the size of the control), independently of the donor. The same treatment regimen was applied to normal mice, and acceleration of wound closure, significant from day 3, leading to a general improvement of 39% at day 10 was observed (Figure 7A3).

In the Type 2 diabetes mouse model (db/db genetic model), results show UCBMNCs-Exo also accelerated wound healing, exhibiting a significant effect at day 2, a 20% improvement at day 10, and a total time of healing of 17 days which is 4 days sooner than the control treatment (Figure 7A4).

These animals were also treated with the active agent of a known product commercialized for the treatment of human diabetic ulcers (PDGF-BB), at the recommended regimen (once per day, maximum concentration recommended). Surprisingly, UCBMNCs-Exo were significantly more efficient, as they start to accelerate wound closure earlier (day 2, compared to day 5), exhibited higher improvement at day 10 (20% compared to 10% with PDGF-BB), and totally closed the wounds sooner (day 17 compared to day 18 with PDGF-BB) (Figure 7A4). Interestingly, when UCBMNC-Exo are applied twice daily in one wound of diabetic mice, wound healing is accelerated in the control distant wound during the first days, suggesting that they also stimulate healing in distant sites (Figure 7C).

Histologic analysis of the wounds indicates that UCBMNCs-Exo treatment increased re-epithelization, stimulated formation of fibrotic tissue, and was associated with increased cell infiltrations, which may be indicative of augmented infiltration of macrophages, essential for wound healing (Figure 7B).

### Example 4. MiR-150-5p enhances fibroblasts survival to ischemia and keratinocyte migration in vitro.

### Experimental Procedure:

For the *in vitro* assays, endothelial cells, fibroblasts and keratinocytes were transfected with miR-150-5p (5 nM, miRIDIAN microRNA Human hsa-miR-150-5p mimic MIMAT0000082, Dharmacon, Lafayette, Colorado, EUA) and Lipofectamine^{®} RNAiMAX transfection reagent (Invitrogen^{™}) in 100 µl of complete medium for 48 h (Cell Survival and Tube formation assay) or 24 h (cell proliferation and scratch assay).

Transfection with only Lipofectamine was used as a negative control. Vascular endothelial growth factor (VEGF, 50 ng/mL) and platelet derived growth factor (PDGF-BB, 50 ng/mL) were used as positive controls.

All the *in vitro* assays were carried out as described for exosomes bioactivity assessment.

### Results:

Our results show that fibroblasts transfected with miR-150-5p have higher survival after 72 h of ischemia than non-transfected cells (Figure 8A). The same was not true for endothelial cells and keratinocytes. We extended our analyses to migration, proliferation and angiogenesis assays. Keratinocytes transfected with miR-150-5p, but not fibroblasts, had improved migration properties, as assessed by a scratch assay. No statistical differences were observed for cells regarding proliferation and angiogenesis.

### Example 5. MiR-150-5p enhances wound healing in vitro,

We evaluated the *in vivo* effect of hsa-miR-150-5p in a non-disease wound-healing model.

### Experimental Procedure:

Six Male C57BL/6 wild-type mice (8 week-old), purchased from Charles River (France) and weighing between 20 and 30 g, were anesthetized and two full-thickness excision of 6 mm diameter each and 5 cm apart were performed with a sterile biopsy punch in the dorsum of each animal.

A single dose of miR-150-5p (5 µM), complexed with DharmaFECT 1(120 µL) siRNA transfection reagent (Dharmacon, Lafayette, Colorado, EUA), was subcutaneously injected to the wound edge (2 × 40 µL) immediately after the wound creation.

A scramble miR (microRNA Mimic Negative Control, Dharmacon, Lafayette, Colorado, EUA) and PBS were used as negative controls.

Animals were kept in individuals cages with food and water ad libitum, and wound area was observed daily during the total period of the experiment. The wound area was traced every day onto acetate paper to follow the rate of wound closure up to 10 days post wounding. The wound size was determined with the ImageJ software (NIH). The data was presented as percentage of original wound (day 0). The animals were sacrificed 10 days post-wounding.

### Results:

Our results showed that wounds treated with hsa-miR-150-5p healed faster than wounds treated with negative miR-control (scramble) and non-treated (PBS) wounds (Figure 8B).

It is interesting to note that hsa-miR-150-5p is a mononuclear specific miR (Li, et al, 2013; Allantaz, et al, 2012; Zhang, et al, 2010). Previous studies have shown that hsa-miR-150-5p targets VEGF and has both anti-angiogenic and pro-angiogenic activities (He, et al, 2016; Yang, et al, 2015; Li, et al, 2013). Furthermore, hsa-miR-150-5p has been also described as being related with endothelial cell migration (Yang, et al, 2015; Zhang, et al, 2010) and cell proliferation and apoptosis (Gu, et al, 2014; Shi, et al, 2007). Our results show for the first time the involvement of hsa-miR-150-5p as a wound healing inductive miRNA.

## Claims

1. A process for preparing an exosome composition comprising exosomes secreted by UCBMNCs, comprising the following steps:
a) Providing isolated umbilical cord blood mononuclear cells (UCBMNCs),
b) Conditioning the UCBMNCs of step a) by exposure to hypoxic conditions of 0.5% O₂, 5% CO₂, during 18h, in a culture medium depleted of foetal bovine serum (0% FBS),
c) separating and isolating the exosomes secreted by the UCBMNCs of the previous step b), and
d) suspending the isolated exosomes of step c) in a suitable carrier.

2. An exosome composition comprising exosomes secreted by umbilical cord blood mononuclear cells (UCBMNCs) prepared by the process of claim 1, wherein at least one of the exosomes contains one or more miRNA selected from a group consisting of:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, and hsa-miR-101-3p, and
- the exosomes exhibit a spherical morphology,
- the exosomes have an average particle size of 100 nm to 200 nm,
- the exosomes have a negative zeta potential of -10mV to -50mV,
- at least one of the exosomes express the markers CD9, CD34, CD81, and/or Hsc70, and
- the exosomes increase the survival rate of endothelial cells and keratinocytes and stimulate wound healing.

3. An exosome composition, according to claim 2, wherein at least one of the exosomes contain one or more miRNA selected from a group consisting of:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, and hsa-miR-146b-5p,
**preferably** the exosomes contain one or more miRNA selected from a group consisting of:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, and hsa-miR-146a-5p,
**preferably** the exosomes contain one or more miRNA selected from a group consisting of:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, and hsa-miR-342-3p,
**preferably** the exosomes contain one or more miRNA selected from a group consisting of:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, and hsa-miR-26a-1-5p,
**preferably** the exosomes contain one or more miRNA selected from a group consisting of:
hsa-miR-150-5p, hsa-miR-16-5p, hsa-miR-142-3p, hsa-miR-223-3p, hsa-let-7g-5p, and hsa-miR-21-5p,
**preferably** the exosomes contain the miRNA hsa-miR-150-5p.

4. A composition according to claim 2 or 3 wherein the concentration of the exosomes in the composition is of 0.01 **µ**g/mL to 10 µg/mL,
**preferably** the concentration of the exosomes in the composition is of 1 **µ**g/mL to 3 µg/mL,
**preferably** the concentration of the exosomes in the composition is of 0.5 µg/mL-1.5 µg/mL,
**even more preferably,** the concentration of the exosomes in the composition is of about 1 µg/mL, 0.01 µg/mL, 0.25 µg/mL, 0.5 µg/mL, 0.75 µg/mL, 1.0 µg/mL, 1.25 µg/mL, 1.5 µg/mL, 1.75 µg/mL, 2.0 µg/mL, 2.25 µg/mL, 2.5 µg/mL, 2.75 µg/mL, 3.0 µg/mL, 5.0 µg/mL, or 10 µg/mL.

5. A composition comprising exosomes secreted by umbilical cord blood mononuclear cells (UCBMNCs), according to any of the claims 2 to 4 for use as a medicament.

6. A composition comprising exosomes secreted by umbilical cord blood mononuclear cells (UCBMNCs), according to any of the claims 2 to 4 for use as a medicament in tissue repair.

7. A composition comprising exosomes secreted by umbilical cord blood mononuclear cells (UCBMNCs), according to any of the claims 2 to 4 for use in skin open wound therapy, such as an incision, a laceration, a tear, an abrasion, an avulsion or a surgical wound.

8. A composition comprising exosomes secreted by umbilical cord blood mononuclear cells (UCBMNCs), according to any of the claims 2 to 4 for use in burn wounds therapy, such as a thermal burn, a chemical burn or a radiation burn.

9. A composition comprising exosomes secreted by umbilical cord blood mononuclear cells (UCBMNCs), according to any of the claims 2 to 4 for use in wound therapy related to a skin condition or skin disease comprising a wound, such as acne, psoriasis, rosacea, dermatitis, eczema, impetigo, intertrigo, or folliculitis.

10. A composition comprising exosomes secreted by umbilical cord blood mononuclear cells (UCBMNCs), according to any of the claims 2 to 4 for use as a medicament in wound therapy such as arterial ulcers, venous ulcers, diabetic ulcers and pressure ulcers, post-surgical ulcer, traumatic ulcer, mouth ulcer, diabetic foot ulcer or corneal ulcer.

11. A product for wound care comprising an exosome composition according to any of the claims 2 to 4, wherein said composition is in a liquid form, in a powder form or in a spray form, in a cream form, hydrogel form, or in a gel form.

## Patentansprüche

1. Ein Verfahren zur Zubereitung einer Exosomenzusammensetzung umfassend Exosome abgesondert mittels UCBMNCn, umfassend die folgenden Schritte:
a) Bereitstellung von isolierten mononukleären Nabelschnurblutzellen (UCBMNCn),
b) Festsetzung der UCBMNCn aus Schritt a) mittels Aussetzung an hypoxische Bedingungen von 0.5% O₂, 5% CO₂, während 18 h, in einem Kulturmittel, dem fötales Rinderserum entzogen wurde (0% FBS)
c) Trennung und Isolierung der Exosome ausgeschieden mittels der UCBMNCn aus dem vorigen Schritt b) und
d) Aufhebung der isolierten Exosomen aus Schritt c) in einem geeigneten Träger.

2. Eine Exosomenzusammensetzung umfassend Exosome abgesondert mittels mononukleären Nabelschnurblutzellen (UCBMNCn), zubereitet mittels dem Verfahren von Anspruch 1, wobei mindestens ein Exosom eine oder mehrere miRNAs auserwählt aus einer Gruppe bestehend aus:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p und hsa-miR-101-3p, und
- die Exosome weisen eine sphärische Morphologie auf,
- die Exosome haben eine durchschnittliche Partikelgröße von 100 nm bis 200 nm,
- die Exosome haben ein negatives Zetapotential von -10mV bis -50mV,
- mindestens eins der Exosome geben die Markierungen CD9, CD34, CD81 und/ oder Hsc70 wieder, und
- die Exosome erhöhen die Überlebungsrate von Endothelzellen und Keratinozyten und fördern die Wundheilung.

3. Eine Exosomenzusammensetzung gemäß Anspruch 2, wobei mindestens eins der Exosomen eine oder mehrere miRNA enthält, auserwählt aus einer Gruppe bestehend aus:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p und hsa-miR-146b-5p,
**vorzugsweise** die Exosome enthalten eine oder mehrere miRNA auserwählt aus einer Gruppe bestehend aus:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p und hsa-miR-146a-5p,
**vorzugsweise** die Exosome enthalten eine oder mehrere miRNA auserwählt aus einer Gruppe bestehend aus:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p und hsa-miR-342-3p,
**vorzugsweise** die Exosome enthalten eine oder mehrere miRNA auserwählt aus einer Gruppe bestehend aus:
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p und hsa-miR-26a-1-5p,
**vorzugsweise** die Exosome enthalten eine oder mehrere miRNA auserwählt aus einer Gruppe bestehend aus:
hsa-miR-150-5p, hsa-miR-16-5p, hsa-miR-142-3p, hsa-miR-223-3p, hsa-let-7g-5p und hsa-miR-21-5p,
**vorzugsweise** die Exosome enthalten die miRNA hsa-miR-150-5p.

4. Eine Zusammensetzung gemäß Anspruch 2 oder 3, wobei der Exosomengehalt in der Zusammensetzung bei 0.01 ug/mL bis 10 ug/mL liegt,
**vorzugsweise** der Exosomengehalt in der Zusammensetzung liegt bei 1 ug/mL bis 3 ug/mL, **vorzugsweise** der Exosomengehalt in der Zusammensetzung liegt bei 0.5 ug/mL bis 1.5 ug/mL,
**noch vorzugsweise** der Exosomengehalt in der Zusammensetzung liegt bei etwa 1 ug/mL, 0.01 ug/mL, 0.25 ug/mL, 0.5 ug/mL, 0.75 ug/mL, 1.0 ug/mL, 1.25 ug/mL, 1.5 ug/mL, 1.75 ug/mL, 2.0 ug/mL, 2.25 ug/mL, 2.5 ug/mL, 2.75 ug/mL, 3.0 ug/mL, 5.0 ug/mL oder 10 ug/mL.

5. Zusammensetzung umfassend Exosome abgesondert mittels mononukleären Nabelschnurblutzellen (UCBMNCn) gemäß einer der Ansprüche 2 bis 4 zur Anwendung als Arzneimittel.

6. Zusammensetzung umfassend Exosome abgesondert mittels mononukleären Nabelschnurblutzellen (UCBMNCn) gemäß einer der Ansprüche 2 bis 4 zur Anwendung als Arzneimittel bei der Gewebeheilung.

7. Zusammensetzung umfassend Exosome abgesondert mittels mononukleären Nabelschnurblutzellen (UCBMNCn) gemäß einer der Ansprüche 2 bis 4 zur Anwendung bei der Behandlung offener Wunden, wie z.Bsp. einem Schnitt, einer Platzwunde, einem Riss, einer Schürfwunde, einer Knochenabsprengung oder einer OP-Wunde.

8. Zusammensetzung umfassend Exosome abgesondert mittels mononukleären Nabelschnurblutzellen (UCBMNCn) gemäß einer der Ansprüche 2 bis 4 zur Anwendung bei der Behandlung von Brandwunden, wie z.Bsp. einer thermischen Verbrennung, einer chemischen Verbrennung oder einer Strahlungsverbrennung.

9. Zusammensetzung umfassend Exosome abgesondert mittels mononukleären Nabelschnurblutzellen (UCBMNCn) gemäß einer der Ansprüche 2 bis 4 zur Anwendung bei der Wundbehandlung im Zusammenhang mit einer Hauterkrankung oder Hautkrankheit umfassend eine Wunde, wie z.Bsp. Akne, Schuppenflechte, Rosazea, Hautentzündung, Hautausschlag, Eiterflechte, Hautwolf oder Haarbalgentzündung.

10. Zusammensetzung umfassend Exosome abgesondert mittels mononukleären Nabelschnurblutzellen (UCBMNCn) gemäß einer der Ansprüche 2 bis 4 zur Anwendung als Arzneimittel bei der Wundbehandlung, wie z.Bsp. Arteriengeschwür, postoperatives Geschwür, traumatisches Geschwür, Mundgeschwür, diabetisches Fußgeschwür oder Hornhautgeschwür.

11. Ein Produkt zur Wundheilung umfassend eine Exosomenzusammensetzung gemäß einer der Ansprüche 2 bis 4, wobei genannte Zusammensetzung in flüssiger Form, in Pulver- oder Sprayform, als Salbe, Hydrogel oder Gel erscheint.

## Revendications

1. Processus de préparation d'une composition d'exosome comprenant des exosomes sécrétés par des UCBMNCs, comprenant les étapes suivantes :
a) Fourniture de cellules mononuclées provenant du sang du cordon ombilical (ou UCBMNCs),
b) Traiter les UCBMNCs de l'étape a) en les exposant aux conditions hypoxiques de 0.5 % de O₂, 5 % de CO₂, pendant 18h, dans une culture moyennement appauvrie de sérum de fœtus de bovin (0 % FBS),
c) Séparation et isolement d'exosomes sécrétés par des UCBMNCs de l'étape b) précédente, et
d) Suspension d'exosomes isolés de l'étape c) dans un transporteur adéquat.

2. Composition d'exosome comprenant des exosomes sécrétés par des cellules mononuclées provenant du sang du cordon ombilical (ou UCBMNCs) préparée selon le processus de la revendication 1, où au moins un des exosomes contient une ou plusieurs miRNA sélectionné(s) dans un groupe qui consiste en ce qui suit :
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, hsa-miR-146b-5p, hsa-miR-106b-5p, hsa-miR-29a-3p, hsa-miR-17-5p, hsa-miR-29b-3p, et hsa-miR-101-3p, et
- les exosomes exhibent une morphologie sphérique,
- les exosomes ont une taille de particule moyenne de 100 nm à 200 nm,
- les exosomes ont un potentiel zêta négatif de -10 mV à - 50 mV,
- au moins un des exosomes exprime les marqueurs CD9, CD34, CD81, et/ou Hsc70, et
- les exosomes augmentent le taux de survie des cellules endothélial et kératinocytes et stimulent la cicatrisation.

3. Composition d'exosome, selon la revendication 2, où au moins un des exosomes contient un ou plusieurs miRNA sélectionné(s) dans un groupe consistant en ce qui suit :
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, hsa-miR-146a-5p, hsa-miR-19a-3p, hsa-miR-15b-5p, hsa-miR-26b-5p, hsa-miR-30d-5p, et hsa-miR-146b-5p,
les exosomes contiennent **de préférence,** un ou plusieurs miRNA sélectionné(s) dans un groupe consistant en ce qui suit :
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, hsa-miR-342-3p, hsa-miR-191-5p, hsa-miR-103a-3p, hsa-miR-15a-5p, hsa-miR-142-5p, et hsa-miR-146a-5p,
les exosomes contiennent **de préférence,** un ou plusieurs miRNA sélectionné(s) dans un groupe consistant en ce qui suit :
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, hsa-miR-26a-1-5p, hsa-miR-20a-5p, hsa-miR-181a-5p, hsa-miR-451a, hsa-miR-23a-3p, et hsa-miR-342-3p,
les exosomes contiennent **de préférence,** un ou plusieurs miRNA sélectionné(s) dans un groupe consistant en ce qui suit :
hsa-miR-150-5p, hsa-miR-16-5p, hsa-miR-142-3p, hsa-miR-223-3p, hsa-let-7g-5p, hsa-miR-21-5p, hsa-let-7f-5p, hsa-miR-19b-3p, hsa-let-7a-5p, et hsa-miR-26a-1-5p,
les exosomes contiennent **de préférence,** un ou plusieurs miRNA sélectionné(s) dans un groupe consistant en ce qui suit :
hsa-miR-150-5p, hsa-miR-16-5p, hsa-miR-142-3p, hsa-miR-223-3p, hsa-let-7g-5p, et hsa-miR-21-5p,
les exosomes contiennent **de préférence,** le miRNA hsa-miR-150-5p.

4. Composition selon les revendications 2 ou 3 où la concentration d'exosomes dans la composition est de 0,01 µg/mL à 10 µg/mL, la concentration d'exosomes dans la composition est **de préférence,** de 1 µg/mL à 3 µg/mL, la concentration des exosomes dans la composition est **de préférence,** de 0,5 µg/mL-1,5 µg/mL, la concentration des exosomes dans la composition est **encore plus préférablement** de près de 1 µg/mL, 0,01 µg/mL, 0,25 µg/mL, 0,5 µg/mL, 0,75 µg/mL, 1,0 µg/mL, 1,25 µg/mL, 1,5 µg/mL, 1,75 µg/mL, 2,0 µg/mL, 2,25 µg/mL, 2,5 µg/mL, 2,75 µg/mL, 3,0 µg/mL, 5,0 µg/mL, ou 10 µg/mL.

5. Composition comprenant des exosomes sécrétés par des cellules mononuclées provenant du sang du cordon ombilical (ou UCBMNCs), selon une quelconque revendication entre 2 et 4 afin d'être utilisée comme médicament.

6. Composition comprenant des exosomes sécrétés par des cellules mononuclées provenant du sang du cordon ombilical (ou UCBMNCs), selon une quelconque revendication entre 2 et 4 afin d'être utilisée comme médicament de réparation des tissus.

7. Composition comprenant des exosomes sécrétés par des cellules mononuclées provenant du sang du cordon ombilical (ou UCBMNCs), selon une quelconque revendication entre 2 et 4 afin d'être utilisée dans la thérapie destinée à lésion cutanée ouverte, telle qu'une incision, une lacération, une déchirure, une écorchure, une avulsion ou une lésion chirurgicale.

8. Composition comprenant des exosomes sécrétés par des cellules mononuclées provenant du sang du cordon ombilical (ou UCBMNCs), selon une quelconque revendication entre 2 et 4 afin d'être utilisée dans une thérapie destinée aux plaies causées par des brûlures, telles que la brûlure thermale, la brûlure chimique ou la brûlure de radiation.

9. Composition comprenant des exosomes sécrétés par des cellules mononuclées provenant du sang du cordon ombilical (ou UCBMNCs), selon une quelconque revendication entre 2 et 4 afin d'être utilisée dans la thérapie destinée aux lésions relatives à une affection de la peau ou à une maladie de la peau, comprenant une lésion, telle que l'acné, le psoriasis, la rosacée, la dermatite, l'eczéma, l'impétigo, l'intertrigo ou la folliculite.

10. Composition comprenant des exosomes sécrétées par des cellules mononuclées provenant du sang du cordon ombilical (ou UCBMNCs), selon une quelconque revendication entre 2 et 4 afin d'être utilisée comme médicament, dans la thérapie destinée aux lésions telles que les ulcères artériels, les ulcères veineux, les ulcères diabétiques et les escarres, l'ulcère post-chirurgical, l'ulcère traumatique, l'ulcère de la bouche, l'ulcère du pied diabétique ou l'ulcère cornéen.

11. Produit pour le soin des lésions comprenant une composition d'exosome selon une quelconque revendication entre 2 et 4, où ladite composition est sous forme liquide, sous forme de poudre ou sous forme de spray, sous forme de crème, sous forme d'hydrogel ou sous forme de gel.
